# EUROPEAN PATENT SPECIFICATION

(11) **EP 4 182 005 B1**
(45) Date of publication and mention of the grant of the patent: **02.07.2025**
(21) Application number: 21843569.1
(22) Date of filing: 16.07.2021
(51) Int. Cl.: A61N 1/02, A61N 1/378, A61N 1/36, A61N 1/04, A61N 1/05, A61N 1/32

(54) **PULSE GENERATOR FOR TRIGEMINAL NERVE STIMULATION**
IMPULSGENERATOR ZUR TRIGEMINALEN NERVENSTIMULATION
GÉNÉRATEUR D'IMPULSIONS POUR STIMULATION DU NERF TRIJUMEAU

(30) Priority: 17.07.2020 US 202063053288 P
(43) Date of publication of application: 24.05.2023
(73) Proprietor: Neurosigma, Inc., Los Angeles, CA 90024 (US)
(72) Inventor: MILLER, Patrick, Los Angeles, CA 90024 (US)
(74) Representative: J A Kemp LLP
(86) International application number: PCT/US2021/042029
(87) International publication number: WO 2022/016086

(56) References cited:
- US-A1- 2014 081 353
- US-A1- 2015 224 317
- US-A1- 2015 224 317
- US-A1- 2017 020 597
- US-A1- 2018 169 423
- US-A1- 2018 169 423
- US-A1- 2019 126 039

## Description

### FIELD

The present disclosure generally relates to external neurostimulator devices and methods of using the same and more particularly relates to external neurostimulator devices configured to stimulate the superficial (cutaneous) sensory branches of trigeminal nerves.

### BACKGROUND

Currently available surgical treatment methods for certain medical disorders, such as epilepsy or other seizure related disorders, may include stimulation of the nervous system by vagus nerve stimulation (VNS), which has been approved by the United States (U.S.) Food and Drug Administration (FDA). In this method, stimulating electrodes are surgically implanted in contact with the vagus nerve as it passes through the neck. In addition to complications related to anesthesia, potential for infection, cost, and other adverse events with VNS, many of the subjects who undergo VNS treatments do not achieve relief, and there is no reliable predictor of good outcomes from the implanted VNS device.

Other approaches to neuromodulation are the focus of on-going research. For example, implantable approaches are also being studied, including deep brain stimulation (DBS) of specific brain regions, and intracranial stimulation of the same via a device which monitors brain activity and delivers stimuli as needed. However, the risks of DBS include infection, hemorrhage, and injury to deep brain structures.

In some clinical situations, electroconvulsive therapy (ECT) and repetitive transcranial magnetic stimulation (rTMS) have been used for neurological and psychiatric conditions.
Traditionally, brain stimulation has been a primary treatment alternative to medications and psychotherapy, and ECT has been the dominant brain stimulation approach since the first part of the 20th century. However, ECT carries risks of memory and other cognitive side effects, considerable cost, and risks of anesthesia.

Many of the above-described methods are invasive and may have considerable costs and side effects. Further, a substantial percentage of patients do not recover from or get adequate lasting relief for the condition or disorder despite multiple trials of pharmaceutical or surgical treatment.

The information included in this Background section of the specification, including any references cited herein and any description or discussion thereof, is included for technical reference purposes only and is not to be regarded as subject matter by which the scope of the invention is to be bound.

US2018/169423 discloses a pulse generator device for trigeminal nerve stimulation.

### SUMMARY

The invention is defined by the independent claim 1.

One aspect of the subject matter of the present disclosure addresses the aforementioned needs by providing a system and device configured to stimulate the trigeminal nerve that is minimally invasive and has reduced side effects in comparison with other neuromodulation approaches.

Disclosed herein is a system for trigeminal nerve stimulation. In one embodiment, the system includes a storage medium, a pulse generator communicatively coupled to the storage medium, a power source coupled to the pulse generator, and at least one electrode communicatively coupled to the pulse generator. The pulse generator includes a microcontroller which executes instructions from the storage medium and the microcontroller is configured to perform at least one of the following operations: produce electrical pulses having defined characteristics, record a log of use and anomalous events, restrict use to a specified individual, interface with electrodes, provide a signal to the specified individual indicating operational conditions and trouble conditions, and provide a signal to the specified individual indicating an end of a treatment period. In some embodiments, the system may further include a power supply or charging station. The power source may be a battery, such as a rechargeable battery.

Disclosed herein is a pulse generator for trigeminal nerve stimulation. In one embodiment, the generator includes a body having a front and back portion and includes at least one electrode channel. The pulse generator also includes a power source. The pulse generator also includes at least one microcontroller executing instructions from a storage medium and the microcontroller is configured to perform at least one of the following operations: produce electrical pulses having defined characteristics, record a log of use and anomalous events, restrict use to a specified individual, interface with electrodes, provide a signal to the specified individual indicating operational conditions and trouble conditions, and provide a signal to the specified individual indicating an end of a treatment period. The pulse generator further includes a display configured to provide a graphical user interface and at least one user control feature configured to allow a user to control at least one operation of the pulse generator. The pulse generator may further include a power inlet port defined in the body. In one embodiment, the body has dimensions of approximately 115 millimeters (mm) (4.5 inches (in)) Height x 69 mm (2.7 in) Width x 27 mm (1.1 in) Depth and a weight of 145 grams (g) (5.1 ounces (oz.)) without a battery. In some embodiments, the power source may be a battery and the body may include at least one battery cavity defined in the back portion of the body that is configured to accept a battery. In one embodiment, the body is plastic, metal alloy or composite material. In one aspect, the microcontroller limits output current and the current is limited to approximately less than 35 Milliamps (mA). In various embodiments, the current output has an upper limit of approximately 10mA, 7mA or 5mA. In some embodiments, the current output has a lower limit of approximately 2.5mA. In some embodiments, the current output is fixed at approximately 5mA. In one embodiment, the microcontroller is configured to deliver (or delivers) a true square-wave charge-balanced output signal or a non-rectangular output signal. In one aspect, the microcontroller produces electrical pulses having the following characteristics: frequency 1- 300 Hertz (Hz), pulse duration 50-500 microseconds (µsec), duty cycle 1-100 percent (%). In one aspect, the electrode channel comprises at least one groove configured to accept at least one projection located at an end of a lead wire of the electrode assembly to form a lock and key configuration. In one aspect, the electrode channel is keyed for a specific electrode assembly.

Disclosed herein is a non-claimed method for operating a pulse generator having a processing device for stimulating at least one cutaneous trigeminal nerve branch using the pulse generator. In one embodiment, the method includes receiving instructions from a storage medium and performing at least one of the following operations: producing electrical pulses having defined characteristics, recording a log of use and anomalous events, restricting use to a specified individual, interfacing with specialized electrodes, providing a signal to the specified individual indicating operational conditions and trouble conditions, and providing a signal to the specified individual indicating an end of a treatment period. In one aspect, the operation of restricting use to a specified individual comprises requiring a patient user to provide a personal identification number (PIN) or a biometric ID to operate the pulse generator. In one aspect, the PIN is a five digit number and the biometric ID is a fingerprint. In one aspect, the operation of producing electrical pulses having defined characteristics is performed by a microcontroller and the characteristics are frequency 1-300 Hz, pulse duration 50-500 µsec, duty cycle 1-100%. In one aspect, the operation of interfacing with electrodes is performed by at least one electrode channel defined in the pulse generator that is keyed to the electrode.

Disclosed herein is a non-claimed computer-readable medium having computer-executable instructions for performing a process for stimulating a branch of a trigeminal nerve. In one embodiment, the instructions include causing a processor device to produce electrical pulses having defined characteristics, record a log of use and anomalous events, restrict use to a specified individual, interface with specified electrodes, provide a signal to the specified individual indicating operational conditions and trouble conditions, and provide a signal to the specified individual indicating an end of a treatment period.

In one or more non-claimed embodiments, a method for trigeminal nerve stimulation comprises producing, by at least one processor of a pulse generator, pulses delivered to an electrode assembly for at least one therapy session for a patient, where the pulses have defined characteristics. The method further comprises logging, by at least one processor of the pulse generator, data from at least one therapy session for the patient, where the data comprises impedance of the electrode assembly connected to the pulse generator during at least one therapy session and current amplitude of the pulses produced during at least one therapy session. The method further comprises determining, by at least one processor of the pulse generator, an average current amplitude by using the data comprising the current amplitude for at least one of at least one therapy session. The method also comprises determining, by at least one processor of the pulse generator, an average therapy impedance by using the data comprising the impedance for at least one of at least one therapy session. Also, the method comprises determining, by at least one processor of the pulse generator, a charge capacity of a battery of the pulse generator prior to beginning a subsequent therapy session for the patient. Further, the method comprises determining, by at least one processor of the pulse generator, whether the charge capacity of the battery of the pulse generator is sufficient to complete the subsequent therapy session for the patient for a designated duration of time by using the average current amplitude and the average therapy impedance.

In one or more non-claimed embodiments, the method further comprises generating, by at least one processor of the pulse generator, an alert to the patient indicating that the charge capacity of the battery of the pulse generator is insufficient to complete the subsequent therapy session for the designated duration of time, when at least one processor of the pulse generator determines that the charge capacity of the battery is insufficient to complete the subsequent therapy session for the patient for the designated duration of time. In at least one embodiment, the alert is a visual alert and/or an audible alert. In some embodiments, the visual alert comprises text and/or an icon.

In one or more non-claimed embodiments, the method further comprises displaying, by a display of the pulse generator, a screen comprising a graphical user interface (GUI). In at least one embodiment, the screen comprises average therapy data for at least one of at least one therapy session for the patient. In some embodiments, the screen comprises a history at least one therapy session for the patient. In one or more embodiments, the screen comprises a history of the current amplitude of the pulses produced during at least one of at least one therapy session for the patient. In at least one embodiment, the screen comprises a history of the impedance of the electrode assembly connected to the pulse generator during at least one of at least one therapy session for the patient.

In at least one non-claimed embodiment, the method further comprises measuring, by at least one processor of the pulse generator, the impedance of the electrode assembly connected to the pulse generator to generate a measured impedance. The method further comprises determining, by at least one processor of the pulse generator, whether the measured impedance is less than a base threshold impedance value. Further, the method comprises generating, by at least one processor of the pulse generator, an alert to the patient indicating that the measured impedance is below the base threshold impedance value, when at least one processor of the pulse generator determines that the measured impedance is less than the base threshold impedance value.

In one or more non-claimed embodiments, the method further comprises measuring, by at least one processor of the pulse generator, the impedance of the electrode assembly connected to the pulse generator to generate a measured impedance. Also, the method comprises determining, by at least one processor of the pulse generator, whether the measured impedance is greater than a maximum threshold impedance value. In addition, the method comprises generating, by at least one processor of the pulse generator, an alert to the patient indicating that the measured impedance is above the maximum threshold impedance value, when at least one processor of the pulse generator determines that the measured impedance is greater than the maximum threshold impedance value.

In one or more embodiments, a pulse generator device for trigeminal nerve stimulation comprises at least one processor configured to: produce pulses delivered to an electrode assembly for at least one therapy session for a patient, where the pulses have defined characteristics; log data from at least one therapy session for the patient, where the data comprises impedance of the electrode assembly connected to the pulse generator during at least one therapy session and current amplitude of the pulses produced during at least one therapy session; determine an average current amplitude by using the data comprising the current amplitude for at least one of at least one therapy session; determine an average therapy impedance by using the data comprising the impedance for at least one of at least one therapy session; determine a charge capacity of a battery of the pulse generator prior to beginning a subsequent therapy session for the patient; and determine whether the charge capacity of the battery of the pulse generator is sufficient to complete the subsequent therapy session for the patient for a designated duration of time by using the average current amplitude and the average therapy impedance. Further, the pulse generator device comprises a display configured to display a screen comprising a graphical user interface (GUI).

In at least one embodiment, at least one processor is further configured to generate an alert to the patient indicating that the charge capacity of the battery of the pulse generator is insufficient to complete the subsequent therapy session for the designated duration of time, when at least one processor determines that the charge capacity of the battery is insufficient to complete the subsequent therapy session for the patient for the designated duration of time. In some embodiments, the alert is a visual alert and/or an audible alert.

In one or more embodiments, the display is a liquid crystal display (LCD) or an organic light-emitting diode (OLED) display. In at least one embodiment, the pulse generator device further comprises at least one button configured for programming the designated duration of time for the therapy session. In one or more embodiments, the pulse generator comprises dimensions such that the pulse generator fits within a hand of a user. In at least one embodiment, the current amplitude of the pulses produced has an upper limit of 8.0 mA.

In at least one embodiment, at least one processor is further configured to restrict use of the pulse generator by requiring a password for the patient. In one or more embodiments, the password for the patient is programmable.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. A more extensive presentation of features, details, utilities, and advantages of the present invention is provided in the following written description of various embodiments of the invention, illustrated in the accompanying drawings, and defined in the appended claims.

### BRIEF DESCRIPTION OF THE DRAWINGS

The present disclosure, both as to its organization and manner of operation, may be understood by reference to the following description, taken in connection with the accompanying drawings, in which:
Figs. 1A and 1B illustrate the location of several branches (nerves) of the trigeminal nerve and the location of the major foramina for the superficial branches of the trigeminal nerve;
Fig. 2A depicts an example of a subject wearing one embodiment of an electrode assembly and pulse generator according to aspects of the present disclosure;
Fig. 2B depicts an example of a subject wearing another embodiment of an electrode assembly and pulse generator according to aspects of the present disclosure;
Fig. 3A is a front perspective view of another embodiment of a pulse generator according to aspects of the present disclosure;
Fig. 3B is a front plan view of the pulse generator of Fig. 3A;
Fig. 3C is a left side view of the pulse generator of Fig. 3A;
Fig. 3D is a right side view of the pulse generator of Fig. 3A;
Fig. 3E-1 is a top perspective view of the pulse generator of Fig. 3A;

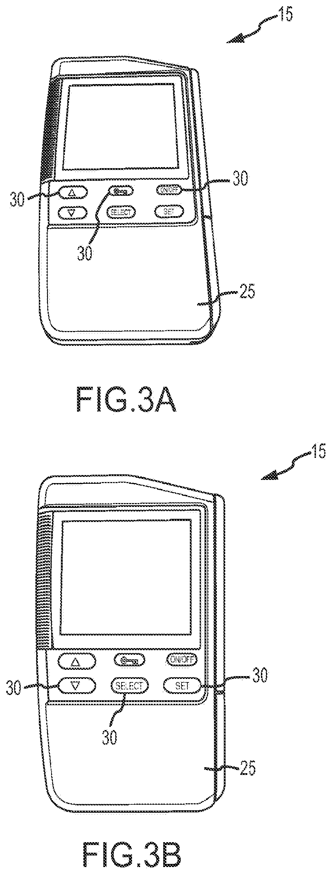

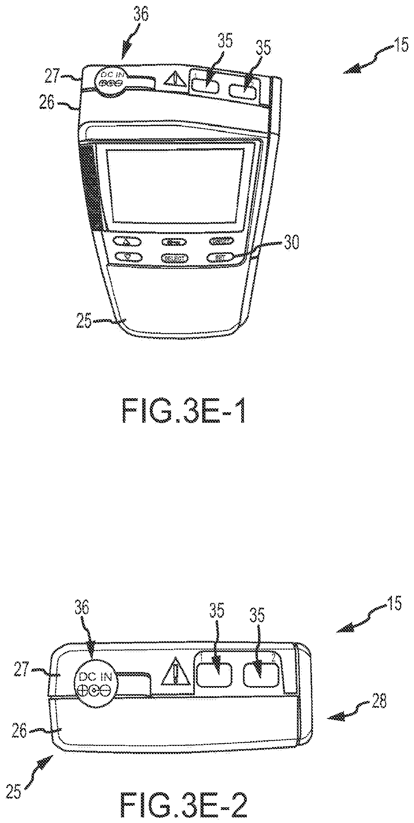

Fig. 3E-2 is a top plan view of the pulse generator of Fig. 3A;
Fig. 3F-1 and Fig. 3F-2 depict top plan views of the inside front and back portions of the housing of the pulse generator of Fig. 3A;

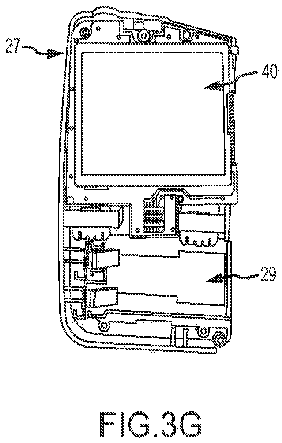

Fig. 3G is a top plan view of the inside back portion of the housing pulse generator of Fig. 3A, wherein some electrical components and a display are shown;

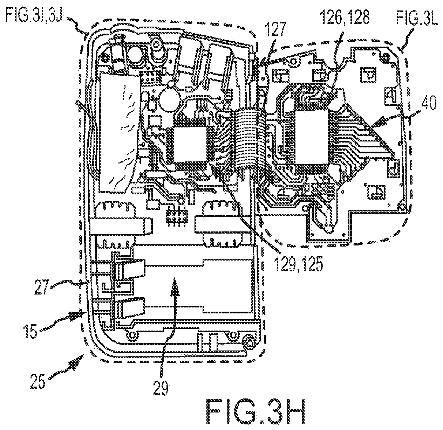

Fig. 3H is the pulse generator of Fig. 3G wherein the electrical components beneath the display are shown;

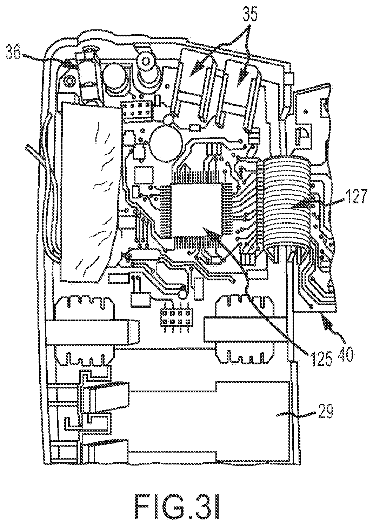

Figs. 3I-3J depict an expanded view of the electrical components shown in Fig. 3H;

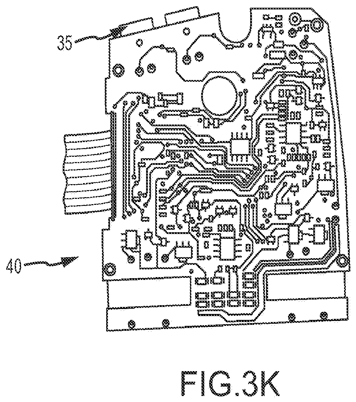

Fig. 3K is a back plan view of the electrical components shown in Figs. 3I-3J;

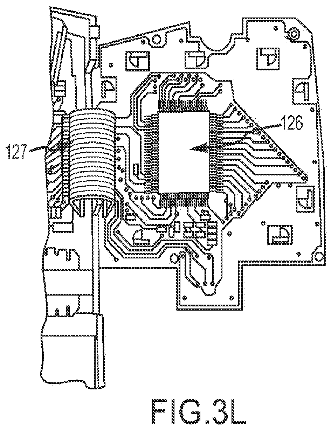

Fig. 3L is a back plan view of the display and associated electrical components of Fig. 3H;

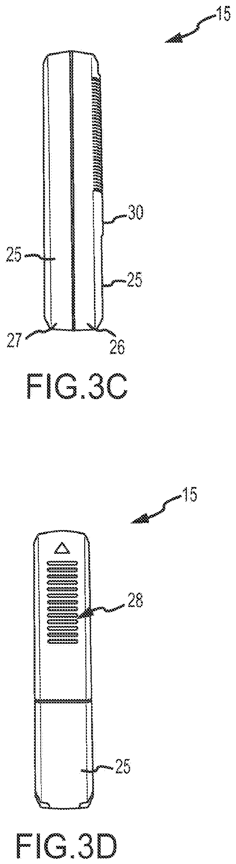

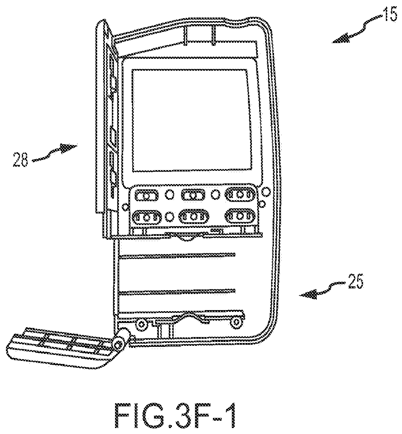

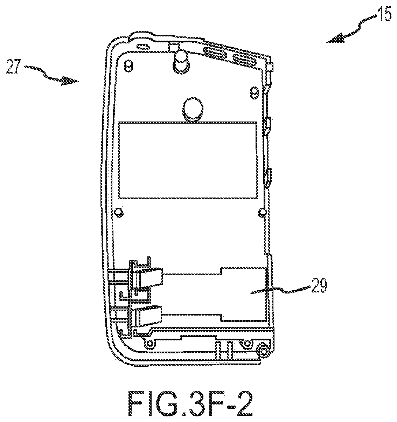

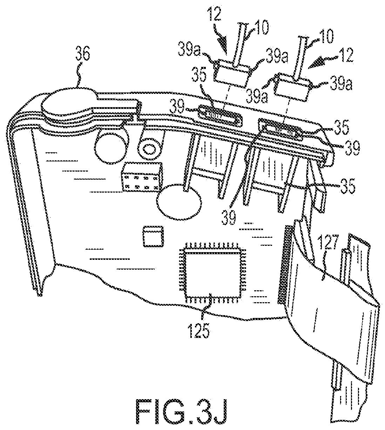

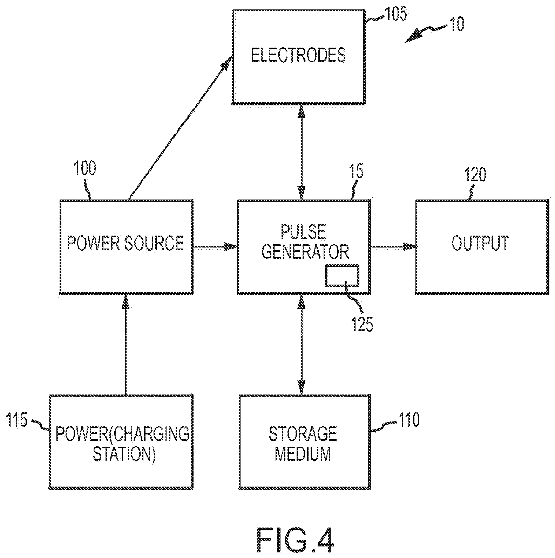

Fig. 4 is a block diagram of the system of Fig. 3;

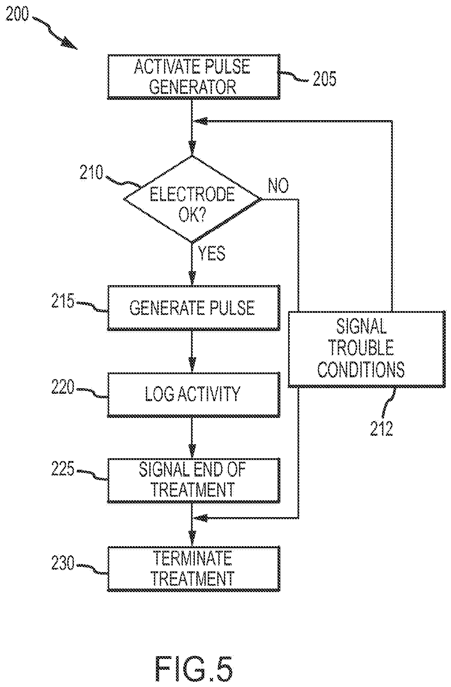

Fig. 5 is a flow chart illustrating one embodiment of a method for operation of a pulse generator;

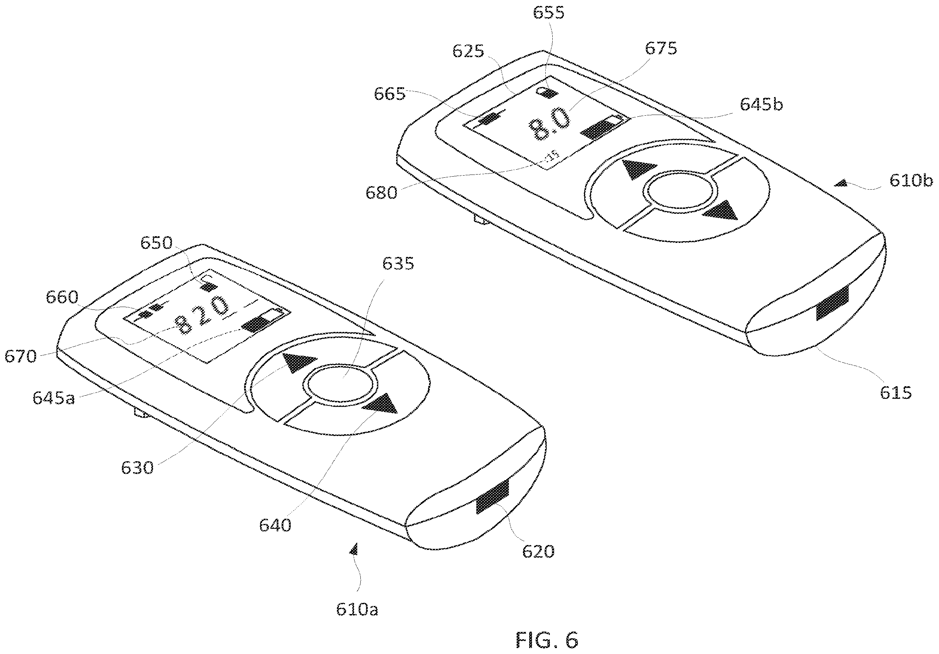

Figs. 6 through 26 are directed to another embodiment of the disclosed pulse generator;
Fig. 6 is a front perspective view of two of the disclosed pulse generators;

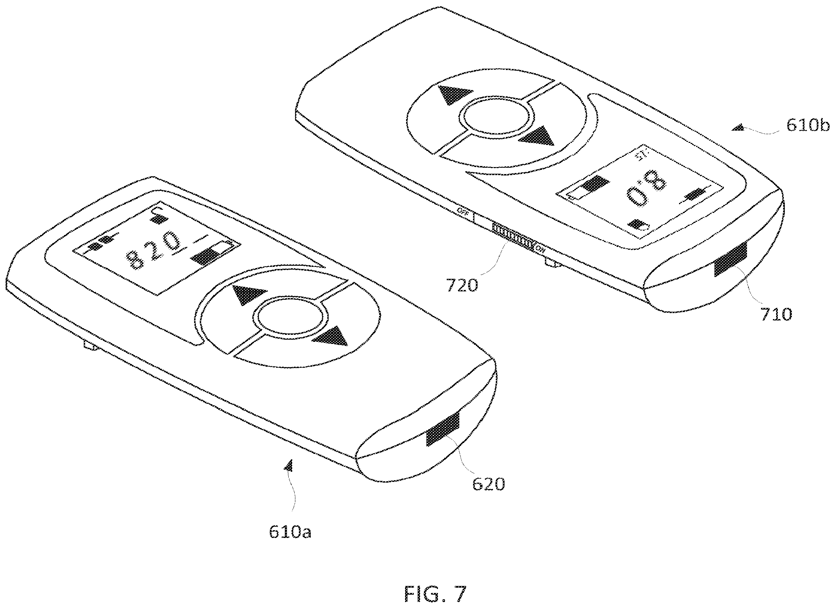

Fig. 7 is another front perspective view of the two pulse generators of Fig. 6;

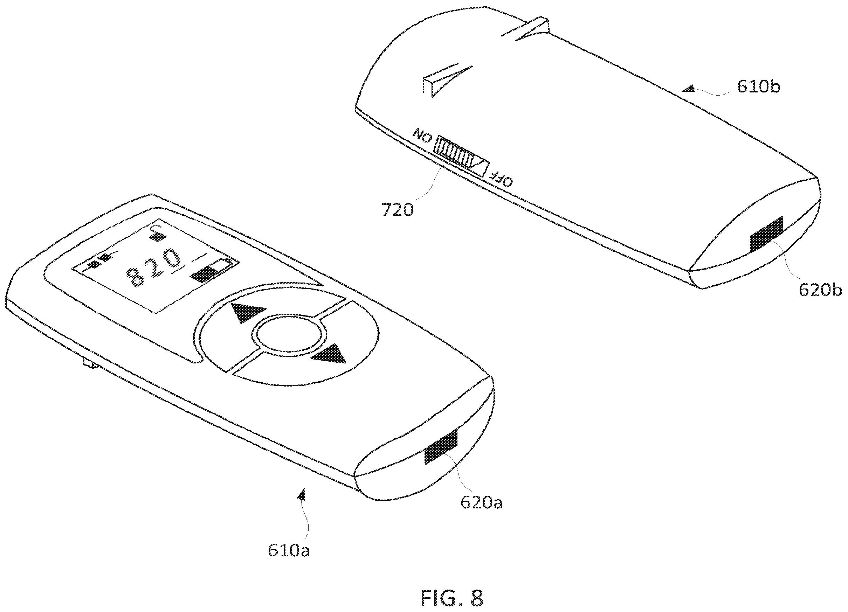

Fig. 8 is a front perspective view of one pulse generator of Fig. 6, and a back perspective view of the other pulse generator of Fig. 6;

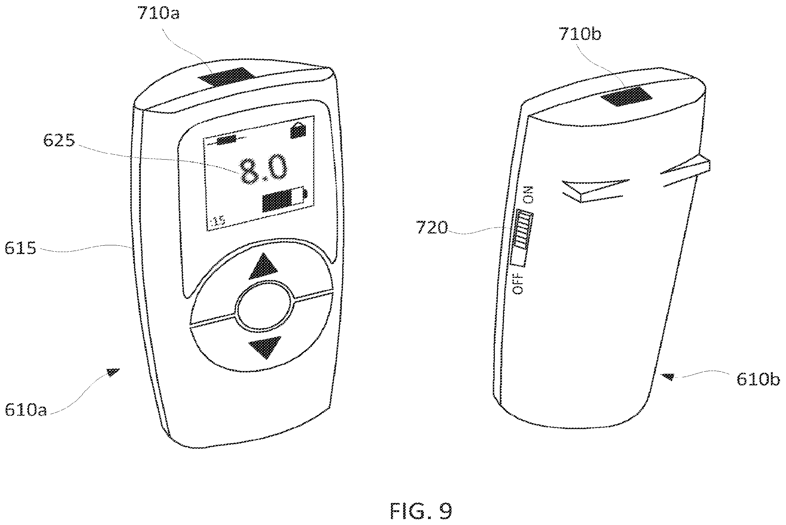

Fig. 9 is a front perspective view of one pulse generator of Fig. 6, and a back perspective view of the other pulse generator of Fig. 6;

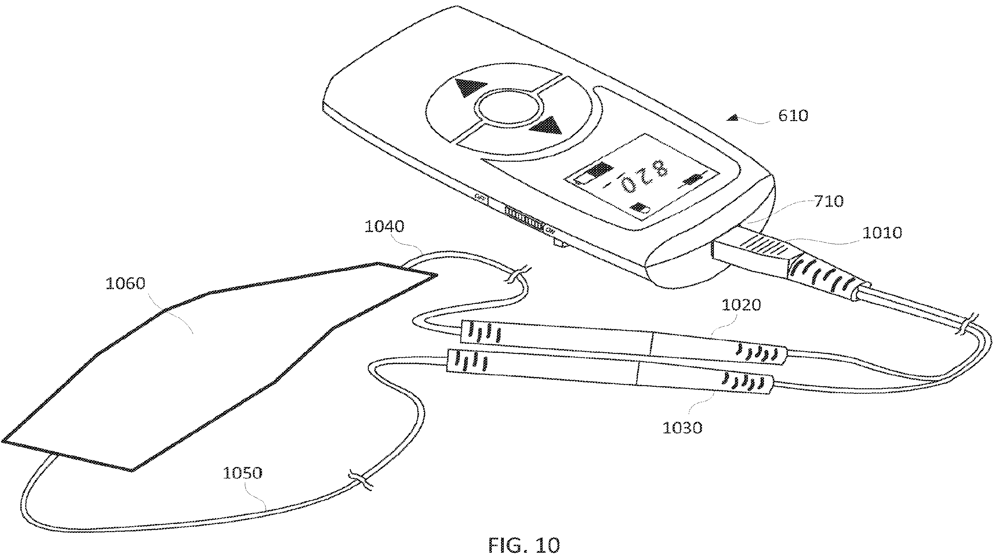

Fig. 10 is a perspective view showing a pulse generator of Fig. 6 connected to an electrode assembly 1060;

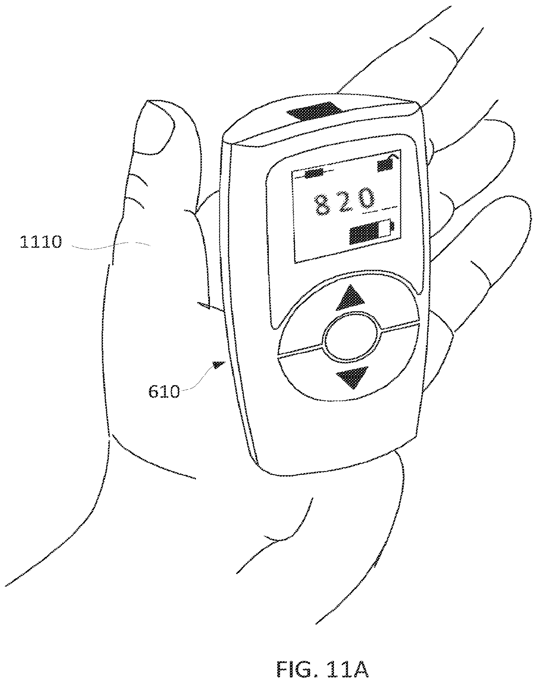

Fig. 11A depicts a user holding a pulse generator of Fig. 6;

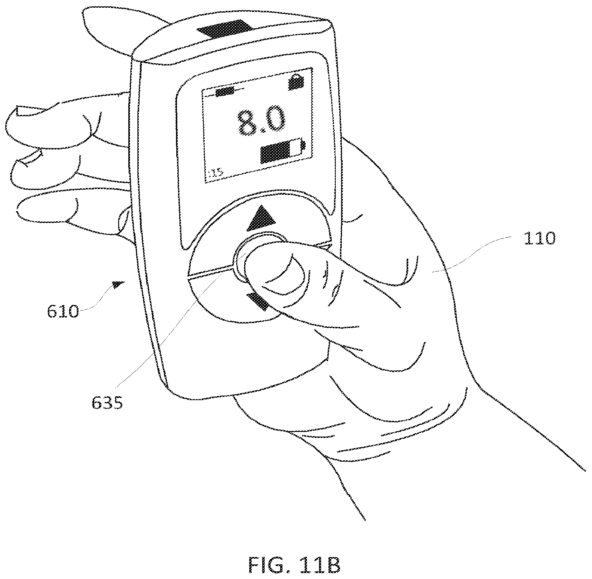

Fig. 11B depicts a user programming a pulse generator of Fig. 6;

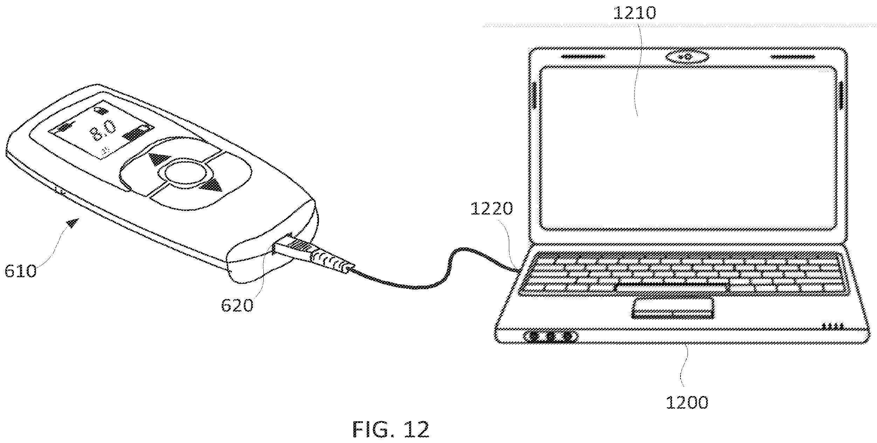

Fig. 12 is a diagram showing a pulse generator of Fig. 6 connected to a computing device;

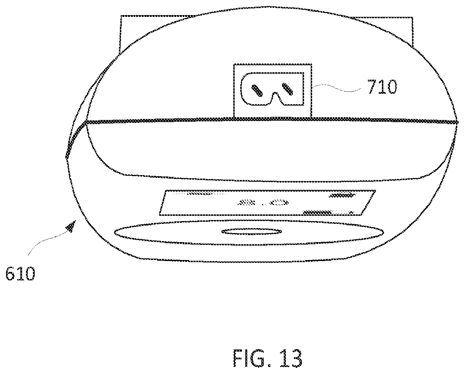

Fig. 13 is a top plan view of a pulse generator of Fig. 6;

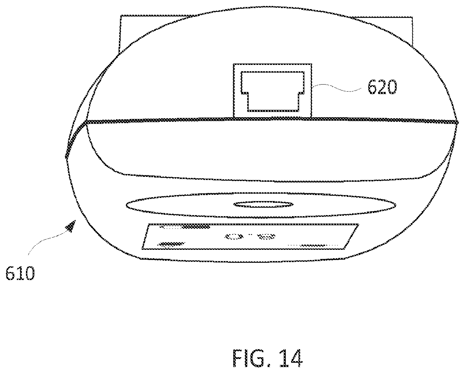

Fig. 14 is a bottom plan view of a pulse generator of Fig. 6;

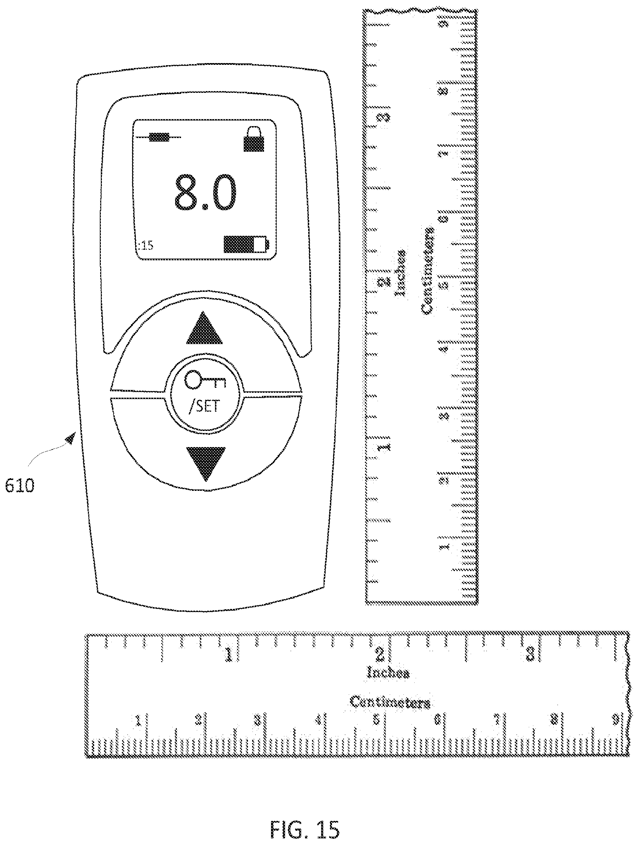

Fig. 15 is a front plan view of a pulse generator of Fig. 6 next to rulers;

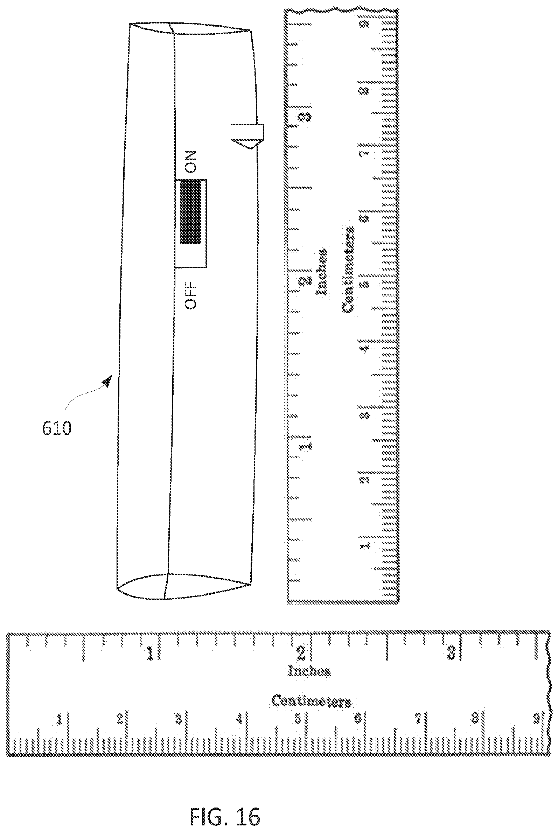

Fig. 16 is a left side view of a pulse generator of Fig. 6 next to rulers;

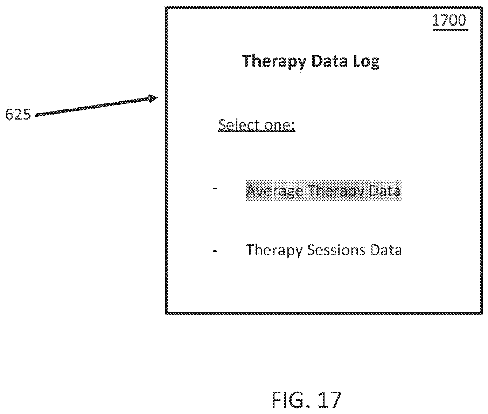

Figs. 17 - 24 show a plurality of exemplary screens that may be displayed on a display of a pulse generator of Fig. 6 to be used by a user to obtain logged therapy data for a patient.
Fig. 17 is a graphical user interface (GUI) screen to access a therapy data log for a patient, where the Average Therapy Data Selection is selected;

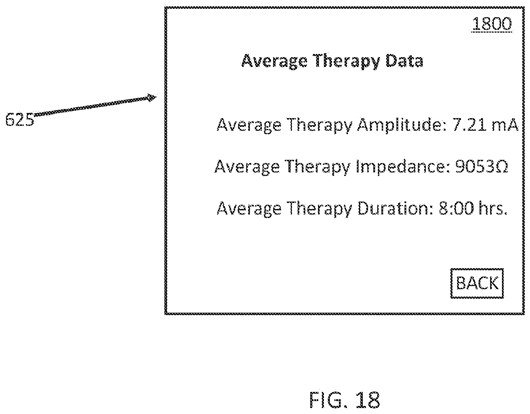

Fig. 18 is a GUI screen showing exemplary average therapy data for a patient;

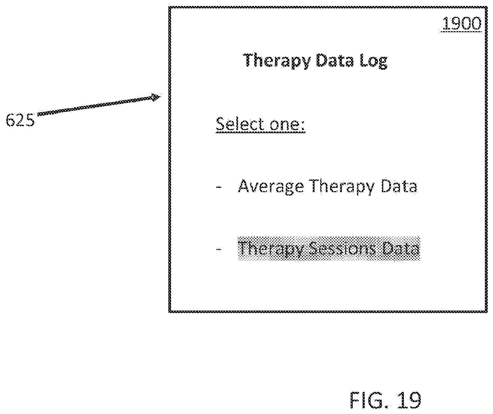

Fig. 19 is a GUI screen to access a therapy data log for a patient, where the Therapy Sessions Data Selection is selected;

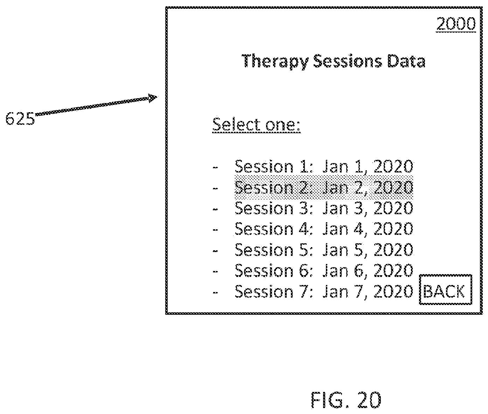

Fig. 20 is a GUI screen showing an exemplary historical listing of therapy sessions for a patient, where Therapy Session 2: Jan 2, 2020, is selected;

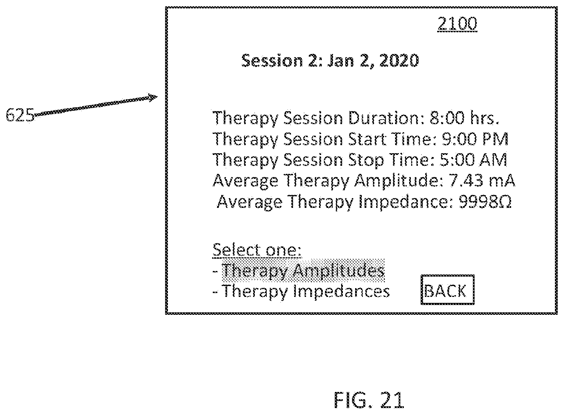

Fig. 21 is a GUI screen showing exemplary therapy data for Therapy Session 2, on January 2, 2020, for a patient, where the Therapy Amplitudes Selection is selected;

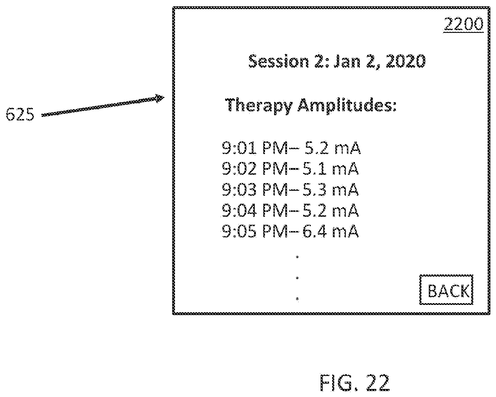

Fig. 22 is a GUI screen showing exemplary historical listing of the therapy amplitudes during Therapy Session 2, on January 2, 2020, for a patient;

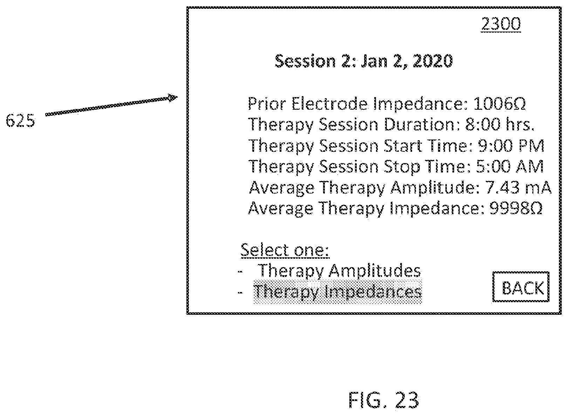

Fig. 23 is a GUI screen showing exemplary therapy data for Therapy Session 2, on January 2, 2020, for a patient, where the Therapy Impedances Selection is selected;

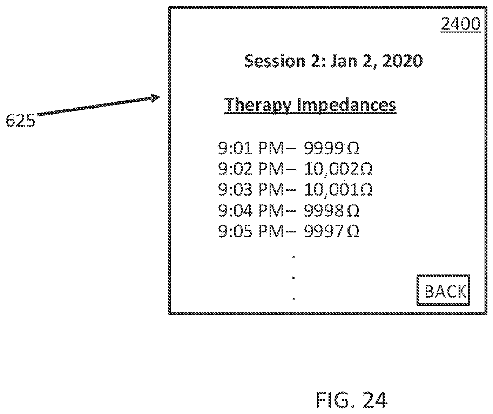

Fig. 24 is a GUI screen showing exemplary historical listing of the therapy impedances during Therapy Session 2, on January 2, 2020, for a patient;

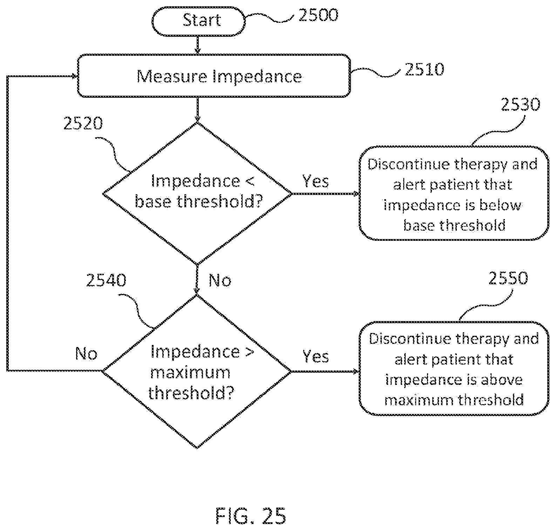

Fig. 25 is a flow chart showing the method for determining whether the impedance of the system is out of range; and

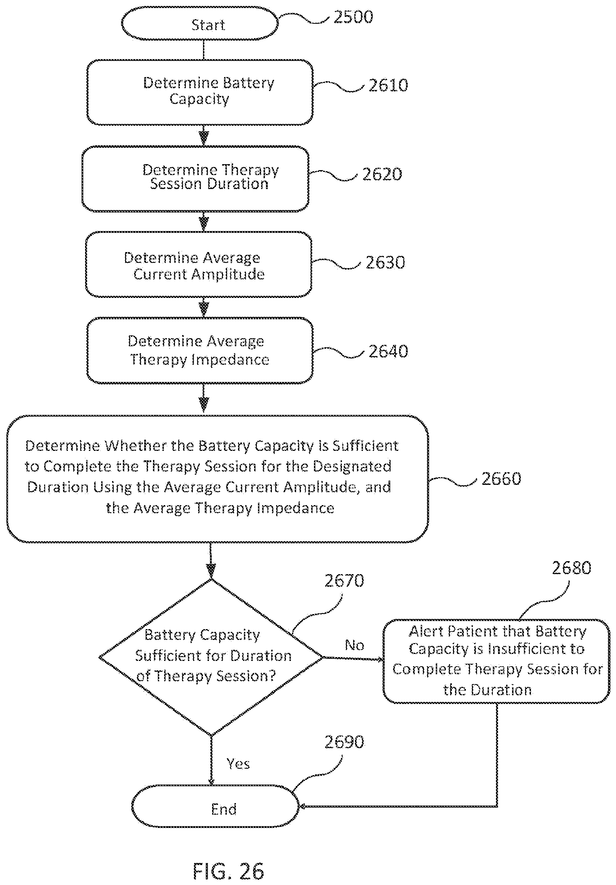

Fig. 26 is a flow chart showing the method for determining whether the battery capacity of a pulse generator of Fig. 6 is sufficient to complete a therapy session for a designated duration.

### DETAILED DESCRIPTION

The present disclosure relates to a device configured for stimulation of the sensory branches of the trigeminal nerve in the face and forehead (trigeminal nerve stimulation or TNS). More specifically, an external pulse generator or neurostimulator configured for stimulation of the sensory components of the ophthalmic nerve and its branches, the infraorbital nerve and its branches, and the mentalis nerves or its branches, and including the supraorbital, supratrochlear, infraorbital, auriculotemporal, zygomaticotemporal, zygomaticoorbital, zygomaticofacial, nasal and infratrochlear nerves is disclosed herein. The pulse generator may be used to treat various disorders, such as neurological disorders as disclosed in, for example, U.S. Patent Application No. 12/898,675, entitled *Systems, Devices and Methods for the Treatment of Neurological Disorders and Conditions* and psychiatric disorders as disclosed in, for example, U.S. Patent Application No.12/898,686 entitled *Devices, Systems and Methods for Treatment of Neuropsychiatric Disorders.*

In previous studies, a commercially available TENS unit, the EMS 7500, has been used. The TENS unit is designed to deliver currents up to 100 mA, well above the levels needed for external TNS. In clinical use for TNS for epilepsy, for example, the high currents would present a potential safety hazard to patients, both for dermal injury and the passage of current through the skull and injuring the brain parenchyma. The pulse generator or neurostimulator as disclosed herein is configured to limit the current delivered and includes a programmable microcontroller to implement the features disclosed herein, thereby reducing the potential for patient injury and optimizing ease of use (user friendliness).

The pulse generator as disclosed herein includes a programmable microcontroller which, in various embodiments, may implement some or all of the following features: produces electrical pulses of specific, programmable characteristics; records a log of use and anomalous events; restricts use to a specified individual; interfaces with various electrode designs, including both subcutaneous implantable electrode designs, such as those described in U.S. application numbers 12/898,685 and 12/898,696, and cutaneous electrode designs, such as those described in U.S. application numbers 12/898,675 and 12/898,686; signals the patient-user (or a physician or other care provider) about operational conditions and trouble conditions; signals the need for a physician reprogramming visit with weekly warning signals and suspending operations until reprogrammed at the specified date; and is rechargeable and is sealed to protect against, for example, liquid penetration into the internal structure of the pulse generator. In some embodiments, the pulse generator may signal a physician or other care provider over the internet or cell phone and the communication may be in real time. In some embodiments, the pulse generator can inform a physician or other care provider that the patient may be having a seizure (based on processing implanted or external EEG data, or other physiologic data such as autonomic nervous system indices (e.g. heart rate variability)) or that the patient has fallen (based on processing data from an accelerometer built into the pulse generator or implanted in the patient).

The unique anatomy of the trigeminal nerve, and its direct and indirect projections to key areas of the brainstem, thalamus and cortex involved with sensory processing, attention, and autonomic function, may allow the use of stimulation by a pulse generator as disclosed herein for a variety of neurological, psychiatric and other conditions in which stimulation may be desirable.

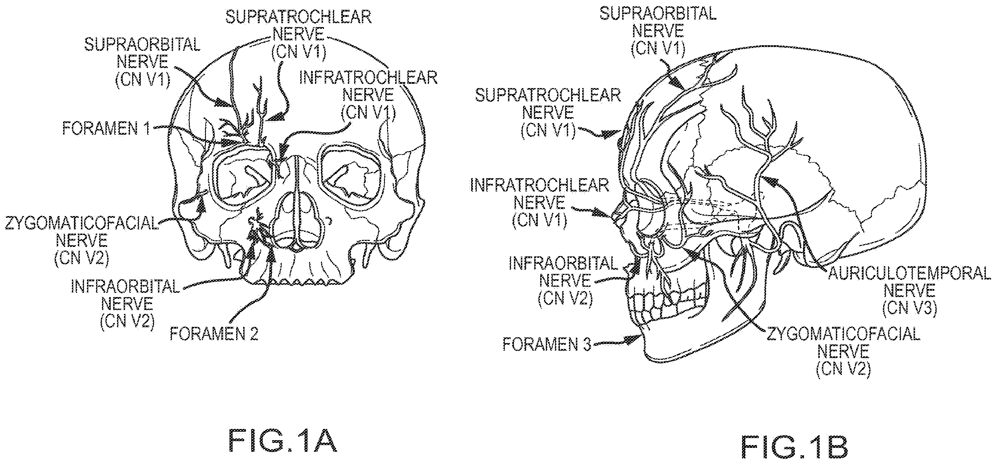

For a discussion related to the trigeminal nerve, reference is now made to Figs. 1A-1B, which illustrate the location of several branches of the trigeminal nerve and the location of the major foramina for the superficial branches of the trigeminal nerve. The trigeminal nerve is the largest cranial nerve and has extensive connections with the brainstem and other brain structures. As it is the fifth of the twelve cranial nerves, it is also known interchangeably as CNV. The trigeminal nerve has three major sensory branches over the face, all of which are bilateral, and highly accessible. The supraorbital nerve, or ophthalmic nerve, is frequently referred to as the V₁ division. The infraorbital branch, or the maxillary nerve, is commonly referred to as the V₂ division. The superficial branch, or the mandibular nerve (also known as the mentalis branch), is referred to as the V₃ division. The supraorbital nerve supplies sensory information about pain, temperature, and light touch to the skin of the forehead, the upper eyelid, the anterior part of the nose, and the eye. The infraorbital branch supplies sensory information about pain, temperature, and light touch sensation to the lower eyelid, cheek, and upper lip. The mentalis branch supplies similar sensory modalities to the jaw, tongue, and lower lip.

As can be understood from FIGS. 1A and 1B, these branches exit the skull through three foramina. The supraorbital nerve or ophthalmic nerve exits at foramen 1 (the supraorbital foramen or notch), approximately 2.1-2.6 cm from the nasal midline (in adults), and is located immediately above the orbital ridge that is near the eyebrow. The nasal nerve is a division of the ophthalmic nerve. The infraorbital branch or maxillary nerve exits at foramen 2 (the infraorbital foramen), approximately 2.4-3.0 cm from the nasal midline (in adults) and the mentalis nerve exits at foramen 3 (the mentalis foramen) approximately 2.0-2.3 cm from the nasal midline (in adults). Other sensory branches, including the zygomaticofacial, zygomaticoorbital, zygomaticotemporal, and auriculotemporal, arise from other foramina.

Fibers from the three major branches join together to form the trigeminal ganglion. From there, fibers ascend into the brainstem at the level of the pons to synapse with the main sensory nucleus of the pons, the mesencephalic nucleus of Cranial Nerve V, and the spinal nucleus and tract of V. Pain fibers descend in the spinal nucleus and tract of V, and then ascend to the ventral posterior medial nucleus (VPM) of the thalamus and then project to the cerebral cortex. Light touch sensory fibers are large myelinated fibers, which ascend to the ventral posterior lateral (VPL) nucleus of the thalamus. Afferent sensory fibers project from the trigeminal nuclei to the thalamus and the cerebral cortex.

The trigeminal nucleus has projections to the nucleus tractus solitarius (NTS), the locus ceruleus, the cerebral cortex, and the vagus nerve. The NTS receives afferents from the vagus nerve and trigeminal nerve. NTS integrates input from multiple sources, and projects to structures in the brainstem and forebrain, including the locus ceruleus.

The locus ceruleus is a paired nuclear structure in the dorsal pons, and is located just beneath the floor of the fourth ventricle. The locus coeruleus has extensive axonal projections to a broad number of brainstem, sub-cortical and cortical structures, and is an important part of the reticular activating system. The locus ceruleus is a core part of the brainstem noradrenergic pathway, and produces the neurotransmitter norepinephrine. Norepinephrine plays a key role in attention, alertness, blood pressure and heart rate regulation, anxiety and mood.

While not wishing to be bound by any particular theory, in certain embodiments, the connections between the trigeminal nerve, locus coeruleus, nucleus and tractus solitarius, thalamus, and cerebral cortex, may be relevant to a potential role of the trigeminal nerve in numerous disorders and conditions. Thus, cutaneous stimulation of the trigeminal nerve via a pulse generator as disclosed herein or a system that includes the pulse generator may be effective in the treatment of multiple disorders and conditions where treatment via trigeminal nerve stimulation is indicated.

Accordingly, stimulation of the superficial or cutaneous branches of the trigeminal nerve provides an avenue for non-invasive neuromodulation. Further, stimulation parameters can be tailored for the individual condition, such that the brainstem, thalamic, or cortical structures involved in the individual condition can be activated or inhibited depending on the pathophysiology of the condition being treated.

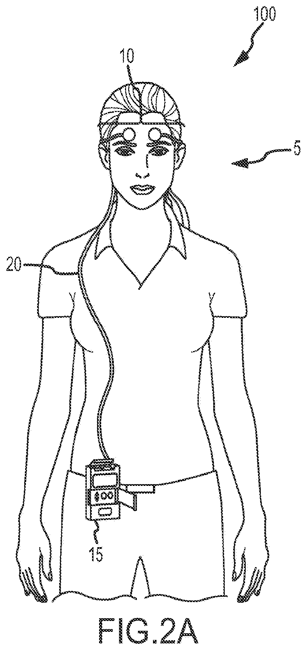

In one embodiment, as can be understood from FIGS. 2A-2B, a system 100 for stimulation of the trigeminal nerve or a branch thereof includes an electrode assembly 10, a neurostimulator or pulse generator 15 and electrical cable or wire 20. The electrode assembly 10 may be configured for the bilateral simultaneous and asynchronous stimulation of the ophthalmic nerves. In other embodiments, the electrode assembly may be configured for unilateral or bilateral stimulation of one or more branches of the trigeminal nerve as disclosed elsewhere herein. The electrode assembly 10 may include a pair of electrodes for placement on a region of the patient's face. It can be appreciated that a single electrode or multiple electrodes may be used. An electrode assembly that may be used with the present disclosure is also described in co-pending US application numbers:12/898,675 and 12/898,686. In one embodiment, the electrical cable or wire 20 is configured to provide a physical and electrical link between the generator 15 and the electrode assembly 10 via lead wires. In other embodiments, the generator 15 and the electrode assembly 10 communicate wirelessly (i.e. the wire 20 and lead wires are not used). In one embodiment, the generator 15 is portable and attached to the belt of the patient 5. In other embodiments, the generator 15 is non-portable. In some embodiments, the system 100 may include a charging station.

In one embodiment, the electrode assembly 10 is configured for bilateral stimulation of both the right and left supraorbital branches of the Trigeminal Nerve (V1) located above the eyebrows over the forehead. The electrode assembly may include both 2-contact and 4-contact electrodes. The contact areas from which the electrical stimulation will travel from the pulse generator to the patient will be placed on the forehead over the V1 branch of the trigeminal nerve bilaterally. The regions of contact are arranged such that the electrical current travels perpendicular to the two branches of the V1 branch between two conductive areas (2 contact) or such that the current travels parallel to the two pathways of the V1 branch (4 contact).

In one embodiment, the electrode assembly 10 may be configured to deliver a symmetric biphasic pulse. In other embodiments, the pulse waveform may be asymmetric and/or multiphasic.

The electrodes may be secured to the forehead by hypoallergenic biocompatible hydrogels, such as DermaFlow^{™} hydrogel (Axelgaard Manufacturing Co, Ltd, Fallbrook, CA, USA). Such gels have been specifically developed for use on the skin and forehead, to minimize skin irritation, and have undergone ISO skin sensitization and histocompatibility studies in animals.

The lead wires 40 carry the electrical impulse from the pulse generator to the conductive regions of contact thereby delivering the prescribed stimulation. In one aspect, the lead wires are 13.5" lead wires that carry the electrical impulse from the pulse generator to the conductive regions of contact. The lead wires exit a single side of the pulse generator and will be bundled together. The lead wires terminate in a specialized plug that connects to the pulse generator's socket, and is configured to prevent a patient-user from connecting the electrodes to other, potentially-hazardous sources of current.

In some embodiments, and as can be understood with reference to Fig. 4, the pulse generator 15 may also be used in conjunction with a physician docking/programming console. In other embodiments, the "programming" functions described herein may be performed directly via the user-interface described elsewhere herein. The programming docking console allows prescribing physicians to set parameters for the user/patient and to monitor the patient's use since last docking event (e.g. by uploading of logfiles). When a patient-user visits the prescribing physician, the pulse generator 15 can be programmed to administer the specific stimulation parameters prescribed by the physician by using the console, such as pulse frequency. These parameters may be set individually, or the physician may select from pre- established combinations (of, e.g., repetition frequency, pulse width, on-period/off-period). The docking/programming station may provide for selection of these parameters from menus or offer stepwise setting of parameters, within ranges and steps as allowed by the pulse generator 15. At follow-up visits, the logfiles may be examined to ascertain actual patterns of device use, as this information may be useful in treatment planning. These data may be displayed as text, or may be presented graphically, for example, as a graph showing amounts of daily use. These data may be stored for the physician to incorporate into the medical record for an individual patient.

For a more detailed discussion of the pulse generator, reference is now made to Figs. 3A-3L, which illustrate various views of one exemplary embodiment of the pulse generator, Fig. 4, which is a block diagram depicting one embodiment of the pulse generator 15, and Fig. 5, which is a flow chart illustrating one embodiment of a method for operation of the pulse generator 15.

As can be understood from Figs. 3A-3L, and with reference to Figs. 4 and 5, the pulse generator 15 comprises a sealed body or case 25 which encloses or houses the internal components, such as the microcontroller and battery discussed below, and other wiring and electronic components. The pulse generator may be manufactured by ITO Co., Ltd, Japan. or other suitable manufacturer.

The sealed body 25 protects the internal components and prevents liquids, etc. from penetrating the body and damaging internal components. In one embodiment, the pulse generator 15 is housed in a rectangular hard plastic case 25 with dimensions of approximately 115mm (4.5in) H x 69mm (2.7in) W x 27mm (1.1in) D and a weight of 145gr (5.1oz) without a battery. In other embodiments, the body or case may be made of a metallic alloy or a composite material. As shown in Fig. 3C, and others, the body 25 includes a front portion 26 and a back portion 27. The front and back portion are sealingly engaged to prevent fluids, etc. from entering the body and interfering with the internal and electrical components housed within the body. As shown in Figs. 3D and 3F-1, the body 25 may include raised features 28 configured to provide a gripping surface by which a user, physician, etc. can open the pulse generator to, for example, replace a battery or other electrical component. As can be seen in Figs. 3A-3B and others, the pulse generator 15 may also include user control features 30, e.g. buttons, that allow the user to turn the power on and off or that provide a temporary lock. In some embodiments, the user control features 30 may be up and down arrow buttons that allow the patient-user to adjust the stimulus amplitude. In some embodiments, this is the only parameter which is user-adjustable (all others are controlled via the physician's programming).

As shown in Figs. 3E-1, 3E-2, 3I-3K, and others, the pulse generator 15 may also include at least one specialized socket or channel 35 for connecting the lead wires of the electrodes. The channel 35 comprises grooves or openings 39 configured to accept an end of the lead wires of the electrode assembly. That is, the channel 39 is "keyed" for the end of the electrode or the electrode assembly. The end of the lead wire 12 includes corresponding projections 39a such that the projections 39a are received in the grooves 39 in a lock and key type configuration. This lock and key configuration prevent a patient-user from connecting the electrodes to other, potentially-hazardous sources of current and other incompatible electrode assemblies from being used with the pulse generator. In use, when a two-contact electrode is used, one channel 35 is utilized. When a four contact electrode is used, two channels 35 are utilized.

As can be seen in at least Figs. 3E-1 and 3E-2, the pulse generator 15 may also include a power inlet port 36. The power inlet port 36 is configured to accept a connector from a power supply source, such as a DC power supply. In use, the pulse generator is powered by a battery and/or the power supply source (not shown).

In some embodiments, the pulse generator 15 is powered by a rechargeable lithium-ion 9V battery that is received in a battery cavity 29 of the body 25, as shown in at least Figs. 3F-2, 3G and 3I. In some embodiments, the generator 15 is powered by lithium-polymer batteries.

As can be understood from Figs. 3G, 3H, 3I, 3K, 3L and others, the pulse generator 15 also includes a display 40, such as an LED or LCD screen, to display a graphical user interface (GUI). The display may also be controlled by a microcontroller 126 on a display PCB 128. The display PCB 128 is coupled to the pulse generator microcontroller 125 on the microcontroller PCB 129 via a ribbon 127. The display 40 may be manipulated by user control features 30 that allow the physician and patient to select specific graphical menus. The user control features 30 may be generated on the GUI or may be features of or integrated with the case 25. The GUI may include a touch-screen interface, thereby allowing the user-patient to make a selection by touching it on the screen.

The GUI is used to control the electric stimulation parameters and, in some embodiments, may provide password protection. In one embodiment, two levels of password protection are provided. The first level of protection allows patients to change their stimulation parameters within a range that has been predetermined by qualified medical personnel, which may be limited to electrical current amplitude. The second level of password protection allows qualified medical personnel to limit the range of stimulation parameters available to the patient. In addition to these parameters, qualified medical personnel can select timed therapy regimens of 1 to 16 hours, as well as a continuous stimulation mode.

As can be understood from Fig. 4, the pulse generator is coupled to a power source 100. With reference to Figs. 3A-3L, in one embodiment, the pulse generator 15 is operably coupled to a battery 100. In other embodiments, the power source may be any suitable power source, such as a fuel cell, or etc. In some embodiments, the battery 100 is rechargeable using inductive coupling to the patient's home base station. In some embodiments, the rechargeable battery has a 5 year life. The battery 100 and/or the pulse generator 25 may be operably coupled to a (an additional) power supply or charging station 115, such as the patient's home base station. The battery may be an internal lithium rechargeable battery. In one embodiment, the battery has a capacity up to 1000 mA-hours to last a minimum of 36-48 hours between charges. In some embodiments, the pulse generator may also be used in conjunction with the patient recharging station. In one embodiment, the patient recharging station is a bedside stand and recharging facility for storing the device when not in use. The pulse generator 15 is also operably coupled to electrodes 105 (which may be a part of the electrode assembly 10). In some embodiments, the pulse generator 15 may be coupled to the electrodes 105 via the wire 20 or the generator 15 and the electrodes 105 may be wirelessly coupled. In some embodiments, the electrodes 105 and the generator 15 may be a single unit, e.g. thegenerator 15 is connected directly to and positioned generally on the electrode. The electrode 105 may be replaced daily (or at another appropriate time) but the generator 15 is reusable. In other embodiments, the generator may be intended for a single-time use (non-reusable). The electrodes 105 may provide data to the pulse generator 15 and the generator 15 may, in turn, produce an output 120, such as notification to the patient that an electrode has become disconnected or that an electrode needs to be repositioned. In some embodiments, the pulse generator 15 may further include a digital display of some or all parameters including output current and skin impedance. As indicated in Fig. 4, the pulse generator 15 is in communication with a storage medium 110. In some embodiments, the storage medium is integrated with the pulse generator. In some embodiments, the storage medium is a separate component of the system. The pulse generator 15 includes a microcontroller 125, or other suitable processor, for receiving and executing instructions from a storage medium 110, such as a non-volatile storage medium, magnetic storage medium, optical storage medium, flash memory, other computer readable medium, or suitable memory device. A processor, such as the microcontroller 125, may control operation of the pulse generator 15. The processor 125 may be any electronic device cable of processing, receiving and/or transmitting instructions. For example, the processor 125 may be a microprocessor, a microcomputer and the like. Various features to be implemented by the programmable microcontroller 125 of the pulse generator 15 are discussed in more detail with reference to Fig. 5.

Fig. 5 is a flow chart illustrating one embodiment of a method 200 for operating a pulse generator in accordance with the present disclosure. The method 200 may be performed by the microcontroller 125, or other suitable processor executing instructions from a computer readable medium. It should be appreciated that the operations of the method 200 may be performed in the order illustrated, in another suitable order and/or one or more operations may be performed simultaneously. Moreover, in some embodiments, the method 200 may include more orfewer operations than those illustrated.

In operation 205, the pulse generator may be turned on or otherwise activated. As part of this operation, the identity of the intended recipient of the treatment may be verified. That is, use of the pulse generator may be restricted to a specific individual patient for which TNS treatment has been prescribed, and may not be used by other unauthorized individuals. In some embodiments, a multi-digit personal code (PIN) that may be chosen by the patient and set by the physician. In some embodiments, the PIN may be a 5 digit code. The patient enters the PIN before treatment will begin. If there are more than a predetermined number of incorrect guesses (e.g. 5) of the PIN, the generator ceases to be operational (e.g. "locks up") for 1 hour (or other appropriate time) and logs the event. In some embodiments, only one treatment session per day is permitted. In other embodiments, a biometric identification system (e.g. a thumbprint) may be used instead of a PIN. The PIN or biometric ID prevent sharing of devices and may reduce the risk of clinically inappropriate use by other individuals.

In operation 210, an electrode check is made. The electrode check may be performed at the start of the session and may monitor the electrode assembly for operational anomalies. In one embodiment, the pulse generator may include a "handshake" with a chip or circuit on or associated with the electrode, which downloads a serial number, and detects the model of electrode (e.g. a single pair of contacts or separate R/L pairs of contacts). In the operation, the pulse generator checks to determine if it is connected to the electrodes, the electrodes are properly positioned and the like. In some embodiments, the pulse generator may further deliver stimulation signals to the electrode contacts within an electrode assembly in this operation and may set the "used" bit on the electrode assembly at the end of treatment to enforce single use. This ensures that the gel on the contacts is uncontaminated, as damaged gel could produce current flow irregularities ("hot spots") leading to skin injury from excessive local current flow. If the electrode check is ok (e.g. the electrodes are connected, properly positioned and the like), then the method may proceed to operation 215-pulse generation.

If the electrode check is not ok, then the method proceeds to operation 212. For example, if impedance suddenly becomes high, then a signal will be sent to indicate that the electrodes have become disconnected ("infinite" impedance). If impedance is low or excessively low, then the user is prompted to relocate the electrodes (e.g. there is a need to reposition the electrode to ensure skin safety). In some embodiments, the signal may also be sent or alternatively sent to a physician or other care provider and/or a designated family member. The pulse generator may signal such trouble conditions. Treatment may be terminated or the patient may adjust the electrodes as indicated and restart treatment (e.g. turning the pulse generator off and then back on or the pulse generator may perform another electrode check).

The method 200 may next proceed to operation 215. A pulse may be generated in this operation. The pulse characteristics may include: (1) controlled-current rectangular pulses, to one or two channels, with one or more of the following characteristics: (a) maximum deliverable current of 30 mA/channel (or as defined elsewhere in this disclosure), (b) physician may set upper and lower bounds for each patient, ranging from 0.3 to 30 mA (e.g. default settings of 1mA lower bound, 20mA upper bound), (c) user-adjustment of actual current delivery within that range to allow setting for comfort, (d) provision for a single bipolar channel, with user ability to swap polarity (e.g. shift from "right side = positive/left side = negative" to the opposite) and (e) provision for a pair of bipolar channels with user ability to swap polarity (e.g. right and left channels are separate pairs, each with a lower and upper electrode contact, and options are "upper pos. / lower neg." and "lower pos. / upper neg." arrangements); (2) pulse width (duration) from 10 to 3000 µs, which may be set by physician (e.g. default 250 µs); (3) repetition rate frequency ranging from 10 to 300 Hz, which may be set by physician; (4) duty cycle adjustable by physician, setting the seconds on and seconds off periods, each variable from 5s to 60s in, for example, 5s steps (e.g. default 30s on / 30s off); and (5) session length of 1 to 23 hours (e.g. default 8 hours). Various embodiments may permit adjustment or programming of any or all of the foregoing. In some embodiments, the operation includes 2-channels, and operates at the following parameters: frequency 1-300 Hz, pulse duration 50-500 µs, duty cycle 1-100%. The two channels may be configured to provide either synchronous or asynchronous stimulation. In some embodiments, the pulse generated in this operation may be transmitted across two separate channels or multiple unique pulses may be generated and carried across a separate channel. In some embodiments, the pulse wave form may be shaped through programmable settings for pulse duration, frequency, and duty cycle and the like. These programmable settings may be adjusted only by a physician or other authorized caregiver in certain embodiments.
Generally, reprogramming of operational parameters discussed herein may be restricted to parties supplying an appropriate password or other credential, such as a biometric indicator. This feature may prevent patients from using the generator at settings contrary to medical prescription or outside of FDA labeling.

As can be understood from the previous section, the programmable microcontroller 125 limits output current. That is, the patient-adjustable current is limited to approximately less than 35 mA to maximize tolerance, minimize current and charge density, and minimize any potential for current penetration through the skull. The controller 125 may deliver true square-wave charge-balanced output. This may be advantageous because existing commercial TENS units have asymmetric output, resulting in irregular stimulation, and risk for formation of hotspots, which may contribute to skin irritation or injury. In some embodiments, asymmetric waveforms may be employed, provided the specific signals are safe.

The microcontroller may be set to a range of outputs. In one embodiment, the range may be set to approximately between 2.5mA and approximately 7mA. In one embodiment, the microcontroller limits the output current to approximately 7 mA, and the patient may adjust the current within a range below 7mA. In another embodiment, the microcontroller can limit the output current to a narrow range (e.g. to ensure safety and compliance) of between approximately 2.5 mA to approximately 5 mA using an external electrode two or four contact electrode. In this way, the patient is prevented from delivering current at too high or too low of an output. In still another embodiment, the output current may be limited to an exact current, e.g. 5 mA, up to a maximum of a fixed current of 7 mA, depending on the size, resistance, or impedance of the electrode. In another embodiment, the output current is limited to a range not to exceed 10 mA, 7 mA, or 5 mA. Without wishing to be limited by any particular theory, it is believed that higher currents, depending on the size and impedance of the electrode, may cause pain, discomfort and/or skin irritation for the patient.

The method 200 may further include an operation 220 in which the activity of the pulse generator is logged. The logging of use operation 220 may include: (1) recording data for each session, such as: (a) session start date-and-time; (b) session stop date-and-time (actual time treatment was ended), (c) user-adjustable setting (e.g. actual current delivered), and (d) session-specific data (e.g. max & min impedance, electrode & configuration). The operation 220 may also include: logging operational anomalies (e.g. electrode disconnects, low impedances, lock-outs for attempts at unauthorized use, etc.), transferring data to the physician's programming console and may include the capacity to store 6 months of treatment data. In other embodiments, less than 6 months or greater than 6 months of data may be stored. A patient's compliance (adherence) and usage is monitored via, e.g., logfiles. Such monitoring may be used to help monitor use patterns in assessing a patient's response to treatment (e.g., a poor clinical response may be linked to using the device less often than prescribed).

In some embodiments, the pulse generator may also signal operational parameters to the patient. This may be part of the logging of use operation 220 or may be part of a differentor a separate operation. For example, a signal may be sent if the user is locked out for PIN guessing, a signal may indicate the minutes until the generator is unlocked. In another example, a signal may be sent if the need for physician follow-up reprogramming is coming up and the signal indicates how many more days of treatment remain before a "refill" date is reached. The pulse generator may also display current time and date in the time zone where programmed, display time left in current session (hr:min) and/or display time needed in charger (hr:min) to be ready for the next session.

The method 200 may further include an operation 225 in which the end of the authorized treatment period is signaled. In one embodiment, several weeks (e.g. default is 3 weeks) prior to the end of the treatment period (e.g. default is 3 months), the operation 230 notifies the user that the treatment period is nearing its end and that a follow up visit with the prescribing doctor for clinical assessment and reprogramming needs to be scheduled. In one embodiment, notification is made at the start of the session. In subsequent weeks, the patient is notified that one less week remains to schedule the visit. In the final week, a daily count-down of days remaining is provided. At the final treatment in the authorized period, the user is notified that this is the last treatment.

In operation 230, the treatment is terminated. Use of the generator may be suspended (e.g. user is locked out) until reprogrammed by a physician or physician programming console or use of the generator may be terminated.

Figs. 6 through 26 are directed to another embodiment of the disclosed pulse generator. In particular, Fig. 6 is a front perspective view of two of the disclosed pulse generators 610a, 610b. It should be noted that the pulse generators 610a, 610b are exact duplicate units, and that the "a" and "b" in the reference numerals in these figures are simply used to indicate the specific pulse generator of the two pulse generators 610a, 610b shown in the figures. In addition, it should be noted that the pulse generators 610a, 610b may each additionally comprise at least some of the features and/or functionality of the pulse generator 15 of Figs. 2A to 4.

In Fig. 6, the pulse generators 610a, 610b are shown to each include a display 625. In one or more embodiments, the display 625 may be a liquid crystal display (LCD) or an organic light-emitting diode (OLED) display. Also in this figure, the pulse generators 610a, 610b are shown to each include a housing 615, which may be a hard-shell housing (e.g., manufactured from plastic).

In addition, the pulse generators 610a, 610b are shown to each include, on their respective bottom side, a port 620. In one or more embodiments, the port 620 is a universal serial bus (USB) port. During operation of the pulse generators 610a, 610b, an end of a cable (e.g., refer to 1230 of Fig. 12) (e.g., a USB cable) is connected to the port 620, and the other end of the cable (e.g., refer to 1230 of Fig. 12) is connected to a computing device (e.g., refer to 1200 of Fig. 12).

Additionally, in Fig. 6, the pulse generators 610a, 610b are shown to each include a plurality of buttons 630, 635, 640. Specifically, button 630 is an up-arrow button, button 640 is a down-arrow button, and button 635 is a lock and set key button. During operation of the pulse generators 610a, 610b, buttons 630, 640 are depressed by a user (e.g., a patient, a care giver (such as a parent), and/or a health care professional (such as a physician)) to scroll up and down, respectively, through text, letters, numbers, characters, icons, and/or screens displayed on the display 625 of the pulse generators 610a, 610b. In addition, during operation of the pulse generators 610a, 610b, buttons 630, 640 are depressed by a user to adjust a current amplitude (up and down) of the pulses generated by the pulse generators 610a, 610b. And, button 635 is depressed by a user (e.g., a patient, a care giver, and/or a health care professional) to select a hi-lighted item (e.g., text, letter, number, character, or icon) displayed on a screen of the display 625. In addition, button 635 may be depressed by a user to lock and unlock the buttons 630, 635, 640 so that they are inactive during a therapy session. It should be noted that the buttons 630, 635, 640 may be locked (e.g., via button 635) such that they are inactive during a therapy session so that the patient may not inadvertently (e.g., during sleep) press any of the buttons 630, 635, 640 during the therapy session.

In Fig. 6, the displays 625 of each of the pulse generators 610a, 610b is shown to display a different screen. In particular, the display 625 of pulse generator 610a is shown to display a login screen, which is used by a user (e.g., a patient, a care giver, and/or a health care professional) to log into the pulse generator 610a to perform a therapy session. On the login screen, a plurality of icons are shown. These icons include an unlocked icon 650, a high impedance signal icon 660, and a battery capacity icon 645a. The unlocked icon 650, when displayed on the display 625, indicates that the buttons 630, 635, 640 of pulse generator 610a are unlocked (i.e. not locked) and are active and available for use by the user. The high impedance signal icon 660, when displayed on the display 625, indicates that the system is exhibiting a high impedance (e.g., an impedance greater than a maximum impedance threshold). Refer to the discussion of Figure 25 for details regarding the determination of a high impedance of the system. The battery capacity icon 645a shows the amount of battery charge remaining in the battery of the pulse generator 610a. In particular, in Fig. 6 the battery capacity icon 645a is showing that the battery has approximately fifty (50) percent (%) of charge remaining. In one or more embodiments, the pulse generator 610a is able to measure the remaining battery capacity to an accuracy of plus or minus (+/-) five (5) %. It should be noted that in one or more embodiments, the pulse generator 610a will switch to a Low Power Mode (e.g., conserving battery capacity) when the battery capacity falls below a predetermined low battery capacity threshold value (e.g., 10 % battery capacity), and/or when an error (e.g., a high impedance) is detected.

Also shown on the display of pulse generator 610a is a password (or a personal identification number (PIN)) for the patient being entered into the pulse generator 610 by the user (e.g., the patient, a care giver, or a health care professional). Specifically, in this figure, the password (or PIN) for the patient comprises four numbers, beginning with "820". The password (or PIN) for the patient may be generated (e.g., by using a random number generator) by the pulse generator 610a for the patient, or may be programmable (e.g., chosen by and customizable) by the user (e.g., the patient, a care giver, or a health care professional) for the patient.

It should be noted that, in one or more embodiments, the password for the patient may comprise (and may be customizable by the user to comprise) more or less than four digits, as is shown in Fig. 6. And, in one or more embodiments, the password for the patient may be programmable to comprise only numbers, only letters (which may be case sensitive), only characters, only icons, or a combination of numbers, letters, characters, and/or icons.

In one or more embodiments, after the user has logged into the pulse generator 610a for the patient, the pulse generator 610a will automatically lock the buttons of the device after a specific duration of time (e.g., 5 minutes) of user inactivity has lapsed.

In Fig. 6, the display of pulse generator 610b is shown to display a therapy session screen, which is used by a user (e.g., a patient, a care giver, and/or a health care professional) to program the parameters for a therapy session for the patient. On the therapy session screen, a plurality of icons are shown. These icons include a locked icon 655, a solid connection symbol icon 665, and a battery capacity icon 645b. The locked icon 655, when displayed on the display 625, indicates that the buttons 630, 635, 640 are unlocked and are active for use by the user. The solid connection symbol icon 665, when displayed on the display 625, indicates that the system is adequately electrically connected (e.g., the impedance of the system lies between a maximum impedance threshold and a base impedance threshold) for a therapy session. The battery capacity icon 645b shows the amount of battery charge remaining in the battery of the pulse generator 610b. In particular, in Fig. 6 the battery capacity icon 645b is showing that the battery has approximately seventy-five (75) % of charge remaining. In one or more embodiments, the pulse generator 610b is able to measure the remaining battery capacity to an accuracy of +/- 5 %. It should be noted that in one or more embodiments, the pulse generator 610b will switch to a Low Power Mode (e.g., conserving battery capacity) when the battery capacity falls below a predetermined low battery capacity threshold value (e.g., 10 % battery capacity). In some embodiments, the pulse generator 610b will prevent the user from initiating a therapy session for a patient when the battery capacity is below a low battery capacity threshold value.

Also on the therapy session screen on the display 625 of pulse generator 610b, the therapy session duration is shown. In particular, in Fig. 6, the therapy session screen on the display 625 of pulse generator 610b shows that the therapy session duration 675 is set by the user (e.g., a patient or health care provider) to be 8.0 hours. Additionally, on the therapy session screen on the display 625 of pulse generator 610b, the therapy progress (time remaining) 680 is shown to be fifteen (15) minutes.

In addition, in one or more embodiments, the therapy session screen on the display 625 of pulse generator 610b may show an impedance indicator (not shown), which indicates the impedance of the electrodes on the electrode assembly (e.g. refer to 1060 of Fig. 10). Also, in one or more embodiments, the therapy session screen on the display 625 of pulse generator 610b may show a therapy completion indicator (not shown), which indicates that the therapy session has completed.

Additionally, it should be noted that in one or more embodiments, the therapy session screen on the display 625 of pulse generator 610b may show, for ease of programming by the user, a plurality of preprogrammed timed-therapy programs (i.e. therapy session programs that already have a therapy duration chosen), which the user may choose from to select to use for the therapy session for the patient.

Fig. 7 is another front perspective view of the two pulse generators 610a, 610b of Fig. 6. In this figure, pulse generator 610b is turned upside down such that the top side of the pulse generator 610b is shown. The top side of the pulse generator 610b is shown to comprise a receptacle 710. During operation of the pulse generator 610b, an end of a lead wire (e.g., refer to 1010 of Fig. 10) is connected to the receptacle 710. The lead wire (e.g., refer to 1010 of Fig. 10) splits into two wires having two remaining ends. The two remaining ends of the lead wire (e.g., refer to 1010 of Fig. 10) are connected to a first lead (e.g., refer to 1040 of Fig. 10) (e.g., a positive lead) and a second lead (e.g., refer to 1050 of Fig. 10) (e.g., a negative lead), respectively, of an electrode assembly (e.g., refer to Fig. 10), which also comprises an electrode pad (e.g., refer to 1060 of FIG. 10).

In addition, the left side of pulse generator 610b is shown to include a power switch 720. The power switch 720 may be switched to the "on" position to turn the pulse generator 610b on, and switched to the "off" position to turn the pulse generator off. In this figure, the power switch 720 is shown to be a slide switch. However, in one or more embodiments, various different types of switches other than a slide switch may be employed for the power switch 720 including, but not limited to, a button switch or a toggle switch.

In one or more embodiments, when power switch 720 is switched to the "off" position, the pulse generator 610b is in an Off Mode. In the Off Mode, no therapy is delivered by the pulse generator 610b, no diagnostics are obtained by the pulse generator 610b, and the pulse generator 610b is unable to communicate with a computing device (e.g., 1200 of Fig. 12). In addition, in the Off Mode, the battery of the pulse generator 610b does not provide any power to the electronics of the pulse generator 610b.

In one or more embodiments, when the power switch 720 is switched to the "on" position, the pulse generator 610b may be operating in a Low Power Mode. When the pulse generator 610b is operating in a Low Power Mode, no therapy is delivered by the pulse generator 610b, no diagnostics are obtained by the pulse generator 610b, and the pulse generator 610b is unable to communicate with a computing device (e.g., 1200 of Fig. 12). In this mode, the battery of the pulse generator 610 provides power to the electronics of the pulse generator 610b.

Fig. 8 is a front perspective view of one pulse generator 610a of Fig. 6, and a back perspective view of the other pulse generator 610b of Fig. 6. In this figure, the bottom side of each of the pulse generators 610a, 610b is shown to comprise a port 620a, 620b (e.g., a USB port). During operation, an end of a cable (e.g., refer to 1230 of Fig. 12) (e.g., a USB cable) is connected to each of the ports 620a, 620b, and the other end of each of the cables (e.g., refer to 1230 of Fig. 12) is connected to a computing device (e.g., refer to 1200 of Fig. 12).

Also in this figure, the back side of pulse generator 610b is shown to be rounded in shape. The rounded shape of the back side of the pulse generator 610b allows for comfort to the user (e.g., a patient or a health care provider), when the user is holding the pulse generator 610b in the user's hand (e.g., refer to Figs. 11A and 11B). As such, the shape of the pulse generator 610b is ergonomically designed such that the pulse generator 610b is easy and comfortable to operate by a user using only a single hand. Additionally, the back side of the pulse generator 610b is shown to comprise a couple of protrusions. The protrusions on the back side of the pulse generator 610b allow for the pulse generator 610b to be stable (e.g., not roll) when the pulse generator 610b is placed on a surface (e.g., a tabletop). It should be noted that in one or more embodiments, the back side of the pulse generator 610b may be manufactured to have more or less than the two protrusions as is shown in the pulse generator 610b in Fig. 9. In addition, the protrusions may be formed to be of different sizes and/or shapes than as the protrusions of the pulse generator 610b as is shown in Fig. 9.

Fig. 9 is a front perspective view of one pulse generator 610a of Fig. 6, and a back perspective view of the other pulse generator 610b of Fig. 6. In this figure, the top side of each of the pulse generators 610a, 610b is shown to comprise a receptacle 710a, 710b. During operation, a lead wire (e.g., refer to 1010 of Fig. 10) is connected to each of the receptacles 710a, 710b.

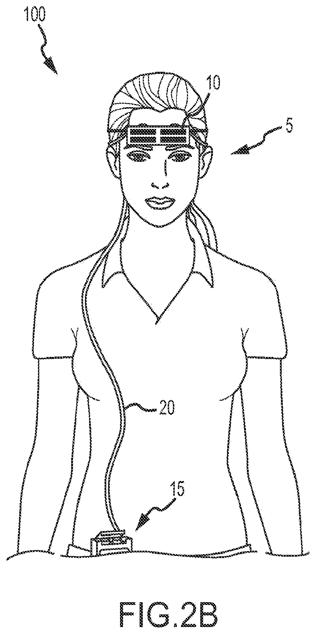

Fig. 10 is a perspective view showing a pulse generator 610 of Fig. 6 connected to an electrode assembly. In this figure, a lead wire cable 1010 is shown to be connected to the receptacle 710 of the pulse generator 610. The lead wire 1010 splits into two wires having two remaining ends. The two remaining ends of the lead wire 1010 are connected to a first lead (e.g., a positive lead) 1040 and a second lead (e.g., a negative lead) 1050, respectively, of an electrode assembly. The electrode assembly comprises the first lead 1040, the second lead 1050, and an electrode pad 1060, which comprises a plurality of electrodes. During operation, the electrode pad 1060 is connected to the head of a patient (e.g., refer to item 10 connected to the forehead of patient 5 of Fig. 2B), and the electrodes of the electrode pad 1060 deliver pulses to the patient.

Fig. 11A depicts a user (e.g., a patient, a care giver, or health care provider) 1110 holding a pulse generator 610 of Fig. 6. As shown in this figure, the pulse generator 610 is ergonomically designed (e.g., designed in size and shape) for single-handed use by a user 1110, and such that the pulse generator 610 fits comfortably in a hand of a user 1110 for use. In one or more embodiments, the pulse generator 610 may be designed to have a volume between two (2) inches cubed (in³) and five (5) in³. In, some embodiments, the pulse generator 610 may be designed to have a volume of approximately 3.7 in³.

Fig. 11B depicts a user (e.g., a patient or health care provider) 1110 programming a pulse generator 610 of Fig. 6. In this figure, the user 1110 is shown to be depressing the button 635 of the pulse generator 610.

As previously mentioned above, the pulse generator 610 comprises a plurality of buttons, which are an up-arrow button 630 (refer to Fig. 6), a down-arrow button 640 (refer to Fig. 6), and a lock and set key button 635. The up-arrow button 630 (refer to Fig. 6) and down- arrow button 640 (refer to Fig. 6) allow for the user 1110 to scroll up and down, respectively, through text, letters, numbers, characters, icons, and/or screens displayed on the display 625 (refer to FIG. 6) of the pulse generator 610. In addition, buttons 630, 640 may be depressed by a user to adjust a current amplitude (up and down, respectively) of the pulses generated by the pulse generator 610. And, the lock and set key button 635 allows for the user 1110 to select a hi-lighted item (e.g., text, letter, number, character, or icon) displayed on a screen of the display 625 of the pulse generator 610.

During operation of the pulse generator 610, prior to initiating a therapy session for a patient, the user 1110 may use (i.e. depress) the up-arrow button 630 (refer to Fig. 6), the down-arrow button 640 (refer to Fig. 6), and/or lock and set key the button 635 to enter the patient's password (or PIN) (refer to 670 of Fig. 6) into the pulse generator 610 to log into the pulse generator for the patient. After the user 1110 has logged into the pulse generator 610 for
the patient, the user 1110 can input the duration for the therapy session for the patient. The user may use (i.e. depress) the up-arrow button 630 (refer to Fig. 6), the down-arrow button 640 (refer to Fig. 6), and/or the lock and set key button 635 to specify the duration for the therapy session.

Once the therapy session begins, the user 1110 may use (i.e. depress) the up-arrow button 630 (refer to Fig. 6), the down-arrow button 640 (refer to Fig. 6), and/or the lock and set key button 635 to select a comfortable current amplitude level for the pulses for the patient. For example, the user 1110 may select a comfortable current amplitude level of 5.2 mA at the beginning of the therapy session.

It should be noted that, in one or more embodiments, the depressing of the up-arrow button 630 (refer to Fig. 6) and/or the down-arrow button 640 (refer to Fig. 6) may adjust the current amplitude level of the pulses by a specific step size (i.e. each depressing of the buttons 630, 640 will change the current amplitude by 0.1 mA). In one or more embodiments, the step size for adjusting the current amplitude level may be 0.1 mA, and the current amplitude level may be adjusted in a range from 0 mA to 8 mA. In one or more embodiments, the step size for adjustment may be greater than or less than 0.1 mA and/or the range of adjustment may vary from the range of 0 mA to 8 mA.

After some time has passed since the beginning of the therapy session, the patient may decide that the initial current amplitude level setting (e.g., of 5.2 mA) is insufficient (e.g., no longer provides any sensation to the patient) or is intolerable and, as such, the patient may decide to increase or decrease the current amplitude level to a higher level or lower level for the pulses. The user 1110 (e.g., the patient or heath care provider) may then again use (i.e. depress) the up-arrow button 630 (refer to Fig. 6), the down-arrow button 640 (refer to Fig. 6), and/or the lock and set key button 635 to select a new comfortable current amplitude level for the pulses for the patient.

During the duration of the therapy session, the pulse generator 610 will log (e.g., refer to 220 of Fig. 5) a history of at least some of the data from the therapy session. In one or more embodiments, during the duration of the therapy session, the pulse generator 610 will log a history of the therapy current amplitudes (e.g., refer to screen 2200 of Fig. 22), and log a history of the therapy impedances (e.g., refer to screen 2400 of Fig. 24). In addition, in one or more embodiments, the pulse generator 610 will log the duration of the therapy session 675 (e.g., 8.0 hours), the average therapy current amplitude for the therapy session, and/or the average therapy impedance for the therapy session. In one or more embodiments, the pulse generator 610 comprises an internal Real Time Clock (RTC), which is used to timestamp all of the logged data.

In one or more embodiments, all of the data collected and logged by the pulse generator 610 during the duration of the therapy session will be stored within the pulse generator 610. A user (e.g., the patient or a health care provider) may access the logged data for a patient in the pulse generator 610 by logging into the pulse generator 610 by using the password (or PIN) 610 for the patient.

Fig. 12 is a diagram showing a pulse generator 610 of Fig. 6 connected to a computing device 1200. In this figure, the pulse generator 610 is connected to the computing device 1200 via a cable 1230. In particular, an end of the cable (e.g., a USB cable) 1230 is connected to a port (e.g., a USB port) 620 of the pulse generator 610. And, another end of the cable 1230 is connected to a port (e.g., a USB port) of the computing device 1200. In this figure, the computing device 1200 is depicted to be a laptop computer comprising a display 1210. However, in other embodiments, the computing device 1200 may be various different types of computing devices other than a laptop computer including, but not limited to, a desktop computer, a server, a smart phone, and a tablet device.

In one or more embodiments, during operation, the computing device 1200 may deliver power to the pulse generator 610 via the cable 1230 to charge the battery of the pulse generator 610. In one or more embodiments, the battery shall charge from twenty-five (25) % capacity to full charge within a period of four (4) hours. In some embodiments, the pulse generator 610 may comprise a light emitting diode (LED) (not shown) to indicate to the user that battery charging is occurring.

In addition, in one or more embodiments, a user may download logged therapy data for a patient from the pulse generator 610 to the computing device 1200 via the cable (e.g., a USB cable) 1230. In some embodiments, the pulse generator 610 will enable connection with the computing device 1200 for the data transfer via the USB connectivity. In at least one embodiment, the pulse generator 610 will communicate (which includes the transmission of the logged data) with the computing device 1200 by using USB protocol.

In some embodiments, the user may run an application (e.g., a data downloading application) (e.g., a personal computer (PC) application) on the computing device 1200 to download logged data for a patient from the pulse generator 610 to the computing device 1200.

In at least one embodiment, the therapy session parameters (e.g., frequency, pulse width, hold time, rest time, and/or ramp time) for the pulse generator 610 may be programmed by a health care provider using the computing device 1200 (e.g., by means of an application running on the computing device 1200).

In one or more embodiments, the pulse generator 610 comprises security means (e.g., password protection, password lock out, firewalls, and/or data encryption) for preventing cybersecurity attacks from other devices. In addition, in some embodiments, the pulse generator 610 utilizes data encryption to protect patient privacy and data.

Fig. 13 is a top plan view of a pulse generator 610 of Fig. 6. In this figure, the top side of the pulse generator 610 is shown to comprise a receptacle 710.

Fig. 14 is a bottom plan view of a pulse generator 610 of Fig. 6. In this figure, the bottom side of the pulse generator 610 is shown to comprise a port 620.

Fig. 15 is a front plan view of a pulse generator 610 of Fig. 6 next to rulers. In this figure, the pulse generator 610 is shown to have an approximate height of 3.5 inches, and an approximate width of 1.5 inches. It should be noted that in other embodiments, the pulse generator 610 may be manufactured to be of various different height and/or width dimensions than as shown in Fig. 15.

Fig. 16 is a left side view of a pulse generator 610 of Fig. 6 next to rulers. In this figure, the pulse generator 610 is shown to have an approximate depth of 0.75 inches. It should be noted that in other embodiments, the pulse generator 610 may be manufactured to be of various different depths than as shown in Fig. 16.

Figs. 17 - 24 show a plurality of exemplary screens that may be displayed on the display 625 of the pulse generator 610 of Fig. 6 to be used by a heath care provider to obtain logged therapy data for a patient. It should be noted that the screens shown in Figs. 17 - 24 are merely example screens that may be employed by the disclosed system, and that the disclosed system may employ various different versions of these exemplary screens and/or may employ more or less screens than as disclosed herein.

Also, it should be noted that, in one or more embodiments, the screens of Figs.17 - 24 may be displayed on the display 1210 (e.g., refer to Fig. 12) of a computing device 1200 (e.g., refer to Fig. 12) to be used by a health care provider to obtain (download) logged therapy data for a patient. In some embodiments, the heath care provider may download the screens of the Figs.17 - 24 via a portable document format (PDF) file.

Fig. 17 is a graphical user interface (GUI) screen 1700 to access a therapy data log for a patient, where the "Average Therapy Data" selection is selected. In this figure, screen 1700 displays two selections for the user to select. In particular, in this figure, the "Average Therapy Data" selection is shown to have been selected by the user. After the "Average Therapy Data" selection is selected by the user, the display 625 will display screen 1800 (refer to Fig. 18).

Fig. 18 is a GUI screen 1800 showing exemplary average therapy data for a patient. In this figure, screen 1800 shows the average therapy data for a patient. In particular, the average therapy data includes the average therapy amplitude, the average therapy impedance, and the average therapy duration. In one or more embodiments, the average therapy data is the average of the data logged for all of the previous therapy sessions for a patient. In some embodiments, a user may specify which of all of the pervious therapy sessions for the patient that the average therapy data may be calculated (i.e. data from only a select number of therapy sessions for the patient will be used for the calculation of the average therapy data).

In one or more embodiments, the screen 1800 comprises a "Back" button, which when selected, will cause the display 625 to display the previous screen (e.g., the Therapy Data Log screen). After the user selects the "Back" button the display will display screen 1900 (refer to Fig. 19).

Fig. 19 is a GUI screen 1900 to access a therapy data log for a patient, where the "Therapy Sessions Data" selection is selected. In this figure, screen 1900 displays two selections for the user to select. In particular, in this figure, the "Therapy Session Data" selection is shown to have been selected by the user. After the "Therapy Sessions Data" selection is selected by the user, the display 625 will display screen 2000 (refer to Fig. 20).

Fig. 20 is a GUI screen 2000 showing an exemplary historical listing of therapy sessions for a patient, where Therapy Session 2: Jan 2, 2020, is selected. In this figure, screen 2000 shows a listing of all of the past therapy sessions for the patient. In particular, in this figure, the "Session 2: Jan 2, 2020" selection is shown to have been selected by the user. After the "Session 2: Jan 2, 2020" selection is selected by the user, the display 625 will display screen 2100 (refer to Fig. 21).

Fig. 21 is a GUI screen 2100 showing exemplary therapy data for Therapy Session 2, on January 2, 2020, for a patient, where the "Therapy Amplitudes" selection is selected. In this figure, screen 2100 shows the therapy session duration, the therapy session start time, the therapy session stop time, the average therapy amplitude, and the average therapy impedance. In addition, the screen 2100 shows two selections for the user to select. In particular, the screen 2100 shows that the user has selected the "Therapy Amplitudes" selection. After the "Therapy Amplitudes" selection is selected by the user, the display 625 will display screen 2200 (refer to Fig. 22).

Fig. 22 is a GUI screen 2200 showing exemplary historical listing of the therapy amplitudes during Therapy Session 2, on January 2, 2020, for a patient. In this figure, screen 2200 shows a listing of all of the therapy amplitudes (which include time stamps) for the patient for this particular therapy session.

In one or more embodiments, the screen 2200 comprises a "Back" button, which when selected, will cause the display 625 to display the previous screen (e.g., the Session 2: Jan 2, 2020 screen). After the user selects the "Back" button the display will display screen 2300 (refer to Fig. 23).

Fig. 23 is a GUI screen 2300 showing exemplary therapy data for Therapy Session 2, on January 2, 2020, for a patient, where the "Therapy Impedances" selection is selected. In this figure, the screen 2300 shows two selections for the user to select. In particular, the screen 2100 shows that the user has selected the "Therapy Impedances" selection. After the "Therapy Impedances" selection is selected by the user, the display 625 will display screen 2400 (refer to Fig. 24).

Fig. 24 is a GUI screen 2400 showing exemplary historical listing of the therapy impedances during Therapy Session 2, on January 2, 2020, for a patient. In this figure, screen 2300 shows a listing of all of the therapy impedances (which include time stamps) for the patient for this particular therapy session.

Fig. 25 is a flow chart showing the method for determining whether the impedance of the system (which comprises the pulse generator connected to the electrode assembly) is out of range. At the start 2500 of the method, at the beginning of the therapy session, at least one processor (e.g., microprocessor 125) of the pulse generator 610 measures the impedance of the system, which comprises the pulse generator connected to the electrode assembly 2510. Then, at least one processor (e.g., microprocessor 125) of the pulse generator 610 determines whether the measured impedance is less than a base threshold value (e.g., 200 Ω), which would indicate a likely short circuit 2520. If at least one processor of the pulse generator 610 determines that the impedance is less than the base threshold value, the pulse generator will discontinue therapy and/or will alert (e.g., by a visual (textual (e.g., ERR) or icon alert and/or an audible alert) the patient that the impedance is below the base threshold value 2530.

However, if at least one processor of the pulse generator 610 determines that the impedance is not less than the base threshold value, at least one processor of the pulse generator 610 will then determine whether the measured impedance is greater than a maximum threshold value (e.g., 15,000 Ω), which would indicate a likely open circuit 2540. If at least one processor of the pulse generator 610 determines that the impedance is greater than the maximum threshold value, the pulse generator will discontinue therapy and/or will alert (e.g., by a visual (textual or icon (e.g., the high impedance signal icon 660 (refer to FIG. 6)) alert and/or an audible alert) the patient that the impedance is above the maximum threshold value 2550.

However, if at least one processor of the pulse generator 610 determines that the impedance is not greater than the maximum threshold value, the method proceeds back to step 2510. Then, the method will continue for the duration of the therapy.

It should be noted that in one or more embodiments, the pulse generator 610 will switch to the Low Power Mode (e.g., conserving battery capacity) any time an error (e.g., the impedance is below a base threshold or the impedance is above a maximum threshold) is detected.

Fig. 26 is a flow chart showing the method for determining whether the battery capacity of a pulse generator 610 of Fig. 6 is sufficient to complete a therapy session for a designated duration. At the start 2600 of the method, at least one processor (e.g., microprocessor 125) of the pulse generator 610 determines (reads) the current battery capacity (e.g., 80 % capacity) 2610. At least one processor of the pulse generator 610 then determines (reads) the duration for the therapy session (e.g., 8.0 hours) 2620. Then, at least one processor of the pulse generator 610 determines (by reviewing logged data for the patient) the average current amplitude of past therapy sessions for the patient 2630. At least one processor of the pulse generator 610 then determines (by reviewing logged data for the patient) the average therapy impedance of past therapy sessions for the patient 2640.

Then, at least one processor of the pulse generator 610 determines whether the battery capacity (e.g., 80 % capacity) is sufficient to complete a therapy session for the designation duration (e.g., 8.0 hours) for the patient by using the average current amplitude and the average therapy impedance 2660.

At least one processor of the pulse generator 610 then determines whether the battery capacity is sufficient for the duration of the therapy session 2670. If at least one processor of the pulse generator 610 determines that the battery capacity is not sufficient for the duration of the therapy session, at least one processor of the pulse generator 610 will alert (e.g., by a visual (textual or icon) alert and/or an audible alert) the patient that the battery capacity is insufficient to complete the therapy session for the duration 2680. In one or more embodiments, Then, if at least one processor of the pulse generator 610 determines that the battery capacity is not sufficient for the duration of the therapy session, at least one processor of the pulse generator 610 will prevent the user from initiating a therapy session at that time. Then, the method ends 2690.

However, if at least one processor of the pulse generator 610 determines that the battery capacity is sufficient for the duration of the therapy session, then the method ends 2690.

In use, in one embodiment, the electrode assembly 10, 1060 is positioned over the forehead of the patient 5. In some embodiments, the electrode assembly 10, 1060 may include an insulative connection region which helps to line up the assembly 10, 1060 with the midline of the nose of the patient 5. In some embodiments, the electrode assembly 10, 1060 is placed over the supraorbital foramina, located over the orbital ridge approximately 2.1-2.6 cm lateral to nasal midline. In one embodiment, the electrode assembly 10, 1060 is then connected to a pulse generator 15, 610 via the electrical cable 20, 1010. In other embodiments, the electrode assembly 10, 1060 is connected to the pulse generator 15, 610 via a wireless connection. In some embodiments, the electrode assembly may be a subcutaneous or percutaneous implantable electrode assembly. In the "percutaneous" form, the electrodes are inserted through the skin but the pulse generator 15, 610 remains external; there may be a lead wire which exits through the skin, or the electrode may be entirely within the skin tissue and they are coupled to a non-implanted pulse generator 15, 610 through, e.g., inductive coupling. The pulse generator 15, 610 then provides stimulation according to methods as described herein.

**As** indicated above, the pulse generator 15, 610 disclosed herein may be used to treat a disorder or condition in a patient using trigeminal nerve stimulation (TNS). Broadly speaking, the method of treatment includes positioning external electrodes over or near at least one of the foramina or branches of the trigeminal nerve (FIG. 1A-1B), and stimulating the electrodes using a stimulator or pulse generator 15, 610 as disclosed herein for a fixed time at specified operational parameters. In one embodiment, the external electrodes are positioned over the foramina of the supraorbital or ophthalmic nerves (FIG. 1A, Foramen 1). In alternative embodiments, the electrode assembly 10, 1060 can be positioned over the foramina of the maxillary nerves (FIG. 1A, Foramen 2) or the mandibular nerves (FIG. 1B, Foramen 3). In yet other embodiments, the stimulation can be unilaterally applied to one foramen of the trigeminal nerves. In other embodiments, electrodes may be positioned at a region of the patient's face (on the right and/or left side) corresponding with the supratrochlear nerve, infratrochlear nerve, zygomaticotemporal, zygomaticofacial, zygomaticoorbital, mentalis, nasal and/or auriculotemporal nerves and/or their respective foramina. In other embodiments, subcutaneous implantable electrodes may be used with the pulse generator 15, 610 as disclosed herein. At least one processor (e.g., programmable microcontroller 125 of Fig. 4) of the pulse generator 15, 610 may be programmed to operate at one or more of the following parameters.

In various embodiments, the stimulation is delivered at a specific pulse width or range of pulse widths (or pulse duration). The stimulation can be set to deliver pulse widths in the range greater than and/or less than one or more of 50 µs, 60 µs, 70 µs, 80 µs, 90 µs, 100 µs, 125 µs, 150 µs, 175 µs, 200 µs, 225 µs, 250 µs, up to 500 µs. Those of skill in the art will recognized that one or more of the above times can be used as a border of a range of pulse widths.

In some embodiments, the stimulation amplitude is delivered as a voltage or current controlled stimulation. In other embodiments it can be delivered as a capacitive discharge. In various embodiments, the current amplitude can be in any range within a lower limit of about 300 µA and an upper limit of about 30 mA - 35 mA, depending on the surface area of the electrodes, inter-electrode distance, the branch(es) stimulated, and the modeling data as described above. In various embodiments, the amplitude can be in a range greater than and/or less than one or more of 50 microamps (µA), 75 µA, 100 µA, 125 µA, 150 µA, 175 µA, 200 µA, 225 µA, 250 µA, 275 µA, 300 µA, 325 µA, 350 µA, 375 µA, 400 µA, 425 µA, 450 µA, 475 µA, 500 µA, 525 µA, 550 µA, 575 µA, 600 µA, 625 µA, 650 µA, 675 µA, 700 µA, 725 µA, 850 µA, 875 µA, 900 µA, 925 µA, 950 µA, 975 µA, 1 mA, 2 mA, 3 mA, 4 mA, 5 mA, 6 mA, 7 mA, 8 mA, 9 mA, 10 mA, 11 mA, 12 mA, 13 mA, 14 mA, 15 mA, 16 mA, 17 mA, 18 mA, 19 mA and 20 mA. In some embodiments, the current amplitudes are less than 7 mA, or less than 6 mA, depending on the size, impedance, resistance, or configuration of the electrode(s). In some embodiments, the current amplitude is between about 2.5mA and about 5 mA. Those of skill in the art will recognize that one or more of the above amplitudes can be used as a border of a range of amplitudes.

In various embodiments, the stimulation can be delivered at one or more frequencies, or within a range of frequencies. The stimulation can be set to be delivered at frequencies less than, and/or greater than one or more of 50 Hz, 45 Hz, 40 Hz, 35 Hz, 30 Hz, 25 Hz, 20 Hz, 15 Hz, or 10 Hz. In various embodiments, the stimulation can be set to be delivered at frequencies greater than, and/or less than, one or more of 20Hz, 30Hz, 40Hz, 50 Hz, 60 Hz, 70 Hz, 80 Hz, 90 Hz, 100 Hz, 120Hz, 125 Hz, 150 Hz, up to 300 Hz. Those of skill in the art will recognize that one or more of the above frequencies can be used as a border of a range of frequencies.

In various embodiments, the stimulation is delivered at a specific duty cycle or range of duty cycles. The stimulation can be set to be delivered at a duty cycle in the range greater than and/or less than one or more of 5%, 10%, 15%, 20%, 25%, 30%, 35%, 40%, 45%, 50%, 55%,
60%, 65%, 70%, 75%, 80%, 85%, 90%, 95%, or 100%. In some embodiments, to ensure preservation of the nerve, a duty cycle of 10% to 50% may be preferable. In some embodiments, duty cycles up to 100% may be useful in particular circumstances. Those of skill in the art will recognize that one or more of the above percentages can be used as a border of a range of duty cycles.

All directional references (e.g., proximal, distal, upper, lower, upward, downward, left, right, lateral, front, back, top, bottom, above, below, vertical, horizontal, clockwise, and counterclockwise) are only used for identification purposes to aid the reader's understanding of the present invention, and do not create limitations, particularly as to the position, orientation, or use of the invention. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. The exemplary drawings are for purposes of illustration only and the dimensions, positions, order, and relative sizes reflected in the drawings attached hereto may vary.

Where non-claimed methods described above indicate certain events occurring in certain order, those of ordinary skill ir the art having the benefit of this disclosure would recognize that the ordering may be modified and that such modifications are in accordance with the variations of the present disclosure. Additionally, parts of methods may be performed concurrently in a parallel process when possible, as well as performed sequentially. In addition, more steps or less steps of the methods may be performed.

The above specification and examples provide a complete description of the structure and use of exemplary embodiments of the invention. Although various embodiments of the invention have been described above with a certain degree of particularity, or with reference to one or more individual embodiments, those skilled in the art could make numerous alterations to the disclosed embodiments without departing from the scope of this invention. Other embodiments are therefore contemplated. It is intended that all matter contained in the above description and shown in the accompanying drawings shall be interpreted as illustrative only of particular embodiments and not limiting. Changes in detail or structure may be made without departing from the basic elements of the invention as defined in the following claims.

## Claims

1. A pulse generator device for trigeminal nerve stimulation, the pulse generator device comprising:
at least one processor configured to:
produce pulses delivered to a cutaneous electrode assembly for at least one therapy session for a patient, wherein the pulses have defined characteristics,
log data from the at least one therapy session for the patient, wherein the data comprises impedance of the cutaneous electrode assembly connected to the pulse generator during the at least one therapy session and current amplitude of the pulses produced during the at least one therapy session,
determine an average current amplitude by using the data comprising the current amplitude for at least one of the at least one therapy session,
determine an average therapy impedance by using the data comprising the impedance for at least one of the at least one therapy session,
determine a charge capacity of a battery of the pulse generator prior to beginning a subsequent therapy session for the patient, and
determine whether the charge capacity of the battery of the pulse generator is sufficient to complete the subsequent therapy session for the patient for a designated duration of time by using the average current amplitude and the average therapy impedance; and
a display configured to display a screen comprising a graphical user interface (GUI).

2. The system of claim 1, wherein the at least one processor is further configured to generate an alert to the patient indicating that the charge capacity of the battery of the pulse generator is insufficient to complete the subsequent therapy session for the designated duration of time, when the at least one processor determines that the charge capacity of the battery is insufficient to complete the subsequent therapy session for the patient for the designated duration of time.

3. The system of claim 2, wherein the alert is at least one of a visual alert or an audible alert.

4. The system of claim 3, wherein the display is one of a liquid crystal display (LCD) or an organic light-emitting diode (OLED) display.

5. The system of claim 1, wherein the pulse generator device further comprises at least one button configured for programming the designated duration of time.

6. The system of claim 1, wherein the pulse generator comprises dimensions such that the pulse generator fits within a hand of a user.

7. The system of claim 1, wherein the current amplitude of the pulses produced has an upper limit of 8.0 mA.

8. The system of claim 1, wherein the at least one processor is further configured to restrict use of the pulse generator by requiring a password for the patient.

9. The system of claim 8, wherein the password for the patient is programmable.

## Patentansprüche

1. Impulsgeneratorvorrichtung für die Stimulation des Trigeminusnervs, wobei die Impulsgeneratorvorrichtung umfasst:
mindestens einen Prozessor, der für Folgendes konfiguriert ist:
Erzeugen von Impulsen, die an eine kutane Elektrodenanordnung für mindestens eine Therapiesitzung für einen Patienten abgegeben werden, wobei die Impulse definierte Eigenschaften haben,
Protokollieren von Daten von der mindestens einen Therapiesitzung für den Patienten, wobei die Daten die Impedanz der mit dem Impulsgenerator verbundenen kutanen Elektrodenanordnung während der mindestens einen Therapiesitzung und die Stromamplitude der während der mindestens einen Therapiesitzung erzeugten Impulse umfassen,
Bestimmen einer durchschnittlichen Stromamplitude unter Verwendung der Daten, die die Stromamplitude für mindestens eine der mindestens einen Therapiesitzung umfassen,
Bestimmen einer durchschnittlichen Therapieimpedanz unter Verwendung der Daten, die die Impedanz für mindestens eine der mindestens einen Therapiesitzung umfassen,
Bestimmen einer Ladekapazität einer Batterie des Impulsgenerators vor dem Beginn einer nachfolgenden Therapiesitzung für den Patienten, und
Bestimmen, ob die Ladekapazität der Batterie des Impulsgenerators ausreicht, um die nachfolgende Therapiesitzung für den Patienten für eine bestimmte Zeitdauer abzuschließen, unter Verwendung der durchschnittlichen Stromamplitude und der durchschnittlichen Therapieimpedanz; und
eine Anzeige, die dafür konfiguriert ist, einen Bildschirm anzuzeigen, der eine grafische Benutzeroberfläche (Graphical User Interface, GUI) umfasst.

2. System nach Anspruch 1, wobei der mindestens eine Prozessor ferner dafür konfiguriert ist, eine Warnung für den Patienten zu erzeugen, die anzeigt, dass die Ladekapazität der Batterie des Impulsgenerators nicht ausreicht, um die nachfolgende Therapiesitzung für die festgelegte Zeitdauer abzuschließen, wenn der mindestens eine Prozessor bestimmt, dass die Ladekapazität der Batterie nicht ausreicht, um die nachfolgende Therapiesitzung für den Patienten für die vorgesehene Zeitdauer abzuschließen.

3. System nach Anspruch 2, wobei die Warnung mindestens eine optische oder akustische Warnung ist.

4. System nach Anspruch 3, wobei die Anzeige eine Flüssigkristallanzeige (Liquid Crystal Display, LCD) oder eine organische Leuchtdiodenanzeige (OLED) ist.

5. System nach Anspruch 1, wobei die Impulsgeneratorvorrichtung mindestens eine Taste umfasst, die zum Programmieren der bestimmten Zeitdauer konfiguriert ist.

6. System nach Anspruch 1, wobei der Impulsgenerator solche Abmessungen umfasst, dass der Impulsgenerator in die Hand eines Benutzers passt.

7. System nach Anspruch 1, wobei die Stromamplitude der erzeugten Impulse eine Obergrenze von 8,0 mA hat.

8. System nach Anspruch 1, wobei der mindestens eine Prozessor ferner dafür konfiguriert ist, die Verwendung des Impulsgenerators einzuschränken, indem er ein Passwort für den Patienten verlangt.

9. System nach Anspruch 8, wobei das Passwort für den Patienten programmierbar ist.

## Revendications

1. Dispositif générateur d'impulsions pour la stimulation du nerf trijumeau, le dispositif générateur d'impulsions comprenant :
au moins un processeur configuré pour :
produire des impulsions fournies à un ensemble d'électrodes cutanées pendant au moins une séance de thérapie d'un patient, les impulsions ayant des caractéristiques définies,
enregistrer des données issues de l'au moins une séance de thérapie du patient, les données comprenant l'impédance de l'ensemble d'électrodes cutanées connecté au générateur d'impulsions pendant l'au moins une séance de thérapie et l'amplitude de courant des impulsions produites pendant l'au moins une séance de thérapie,
déterminer une amplitude de courant moyenne au moyen des données, dont l'amplitude de courant, pour au moins une de l'au moins une séance de thérapie,
déterminer une impédance de thérapie moyenne au moyen des données, dont l'impédance, pour au moins une de l'au moins une séance de thérapie,
déterminer une capacité de charge d'une batterie du générateur d'impulsions avant d'entamer une séance de thérapie subséquente du patient, et
déterminer si la capacité de charge de la batterie du générateur d'impulsions est suffisante pour terminer la séance de thérapie subséquente du patient eu égard à une durée assignée au moyen de l'amplitude de courant moyenne et de l'impédance de thérapie moyenne ; et
un dispositif affichage configuré pour afficher un écran comprenant une interface utilisateur graphique (GUI, graphical user interface).

2. Système selon la revendication 1, dans lequel l'au moins un processeur est configuré en outre pour générer une alerte destinée au patient, indiquant que la capacité de charge de la batterie du générateur d'impulsions est insuffisante pour terminer la séance de thérapie subséquente eu égard à la durée assignée, quand l'au moins un processeur détermine que la capacité de charge de la batterie est insuffisante pour terminer la séance de thérapie subséquente eu égard à la durée assignée.

3. Système selon la revendication 2, dans lequel l'alerte est une alerte visuelle et/ou une alerte sonore.

4. Système selon la revendication 3, dans lequel le dispositif d'affichage est un écran à cristaux liquides (LCD) ou un écran à diodes électroluminescentes organiques (OLED).

5. Système selon la revendication 1, dans lequel le dispositif générateur d'impulsions comprend en outre au moins un bouton configuré pour programmer la durée assignée.

6. Système selon la revendication 1, dans lequel le générateur d'impulsions présente des dimensions lui permettant de tenir dans la main d'un utilisateur.

7. Système selon la revendication 1, dans lequel l'amplitude de courant des impulsions produites a une limite supérieure de 8,0 mA.

8. Système selon la revendication 1, dans lequel l'au moins un processeur est configuré en outre pour restreindre l'utilisation du générateur d'impulsions en demandant un mot de passe pour le patient.

9. Système selon la revendication 8, dans lequel le mot de passe pour le patient est programmable.
